# EUROPÄISCHE PATENTSCHRIFT

(11) **EP 1 393 697 B1**
(45) Veröffentlichungstag und Bekanntmachung des Hinweises auf die Patenterteilung: **15.02.2006**
(21) Anmeldenummer: 02019470.0
(22) Anmeldetag: 30.08.2002
(51) Int. Cl.: A61F 2/46

(54) **Operationssystem**
Operating system
Système d'opération

(43) Veröffentlichungstag der Anmeldung: 03.03.2004
(73) Patentinhaber: Zimmer GmbH, 8404 Winterthur (CH)
(72) Erfinder: Forrer, Michael, 8472 Seuzach (CH); Willi, Thomas, 8484 Weisslingen (CH); Wendt, Peter, 8542 Wiesendangen (CH)
(74) Vertreter: Manitz, Finsterwald & Partner GbR

(56) Entgegenhaltungen:
- US-A- 5 741 335

## Beschreibung

Die Erfindung betrifft ein Operationssystem für eine zwei zusammenwirkende Lagerkörper, insbesondere einen Gelenkkopf und eine Gelenkschale, einen Schaft und eine Kupplung zum Verbinden des Schaftes mit einem der Lagerkörper aufweisende Gelenkprothese, insbesondere Schultergelenkprothese.

Aus der FR-A-2 727 857 (siehe auch US-A-5 741 335) ist eine Schaftprothese für ein Schultergelenk bekannt, die aus einem Stiel oder Schaft und einem Prothesenkopf (Gelenkkopf) besteht, die über ein festsetzbares Kugelgelenk miteinander verbunden sind. Der Prothesenkopf besteht aus einem flachen Kugelausschnitt, dessen ebene Unterseite auf einer vorbereiteten ebenen Oberfläche (Resektionsfläche) des Knochens aufliegen soll, um die Resektionsfläche vollständig abzuschließen. Ein vom Schaft der Prothese in einer schrägen Richtung zur Schaftachse wegstehender Kugelkörper ist durch Schlitze in Lappen aufgeteilt, die durch einen Dorn, der in der schrägen Richtung durch den Schaft hindurch in den Kugelkörper hineingetrieben wird, aufspreizbar sind. Im Prothesenkopf ist eine kugelförmige Lagerschale von der Unterseite eingearbeitet, die den Kugelkörper beim Aufspreizen umfängt und in einer wählbaren Neigung zur Schaftachse blockiert. Bei dieser Prothese besteht ein Nachteil darin, dass die Einstellung der Neigung im Voraus erfolgen muss und dem Operateur ein großes räumliches Vorstellungsvermögen abverlangt. Bei allen Vorteilen, die eine derartige Prothese dennoch bietet, ist außerdem problematisch, dass der Operateur letztlich nur einen einzigen Versuch hat, um eine in Verbindung mit dieser Prothese verwendete Kupplung definitiv festzusetzen.

Um bei einer derartigen Schaftprothese eine genaue Voreinstellung zu erreichen, ist ein Baukasten mit einer Montagevorrichtung zum Montieren von Schaftprothesen vorgeschlagen worden, der in der EP 0 931 522 A1 beschrieben ist. Der Baukasten umfasst Schaftprothesen, die aus verschieden großen Schäften sowie aus verschieden großen Prothesenköpfen zusammengesetzt werden können, wobei zwischen Schaft und Prothesenkopf eine festsetzbare Kupplung angeordnet ist, die unterschiedliche Positionen und Winkellagen zwischen Prothesenkopf und Schaft zulässt. Ferner sind so genannte Probierprothesen vorgesehen, die analog zu den Schaftprothesen in unterschiedlichen Größen zusammengesetzt werden können und eine lösbar festsetzbare Kupplung aufweisen, die bei der im Knochen eingesetzten Probierprothese festsetzbar ist, um die optimale Position und Winkellage des Kopfes der Probierprothese relativ zu deren Schaft festzuhalten. Nach dem Entfernen der Probierprothese werden mit dieser in einem Kopiervorgang die eingestellte optimale Position und Winkellage auf die Montagevorrichtung übertragen, um einen der Probierprothese entsprechenden Schaft und Prothesenkopf in der Montagevorrichtung in der Position und Winkellage der Probierprothese relativ zueinander festzusetzen.

Ein Nachteil dieses bekannten Systems besteht darin, dass mit den Probierprothesen und der Montagevorrichtung ein erheblicher Materialaufwand betrieben werden muss. Ferner ist insbesondere aufgrund des erläuterten Kopiervorgangs eine vergleichsweise lange Operationszeit erforderlich.

Eine weiterentwickelte Schultergelenkprothese, bei der zum Einsetzen zwar immer noch mit der erwähnten Montagevorrichtung gearbeitet wird, um die mit einer Probierprothese ermittelte optimale Stellung eines Probierlagerkopfes relativ zum Schaft auf die eigentliche Prothese zu kopieren, die jedoch für eine bessere Verbindung zwischen Lagerkopf und Schaft sorgt, ist in der noch nicht veröffentlichten EP 01 811 120.3 beschrieben. Hierbei erfolgt die Verbindung zum Schaft durch einen nicht rotationssymmetrischen konischen Körper mit einem selbsthemmenden Sitz, wobei der Umfang des konischen Körpers einen zu seiner Längsachse drehfesten und durch die Konizität verkeilten Formschluss mit einer entsprechend geformten Aufnahme im Schaft bildet, wodurch die Verbindung lösbar und in gleicher Winkellage wiederholt festgesetzt werden kann.

Aufgabe der Erfindung ist es, eine Möglichkeit zu schaffen, Gelenkprothesen auf möglichst einfache und schnelle Weise einzusetzen und dabei eine möglichst große Sicherheit für das Erreichen der jeweils erforderlichen Relativlage zwischen Gelenkkopf und Schaft zu gewährleisten, wobei dies insbesondere unter Beibehaltung der vorhandenen Gelenkprothesen möglich sein soll.

Die Lösung dieser Aufgabe erfolgt durch die Merkmale des Anspruchs 1 und insbesondere dadurch, dass ausgehend von dem eingangs erwähnten Operationssystem eine Positioniereinrichtung, mittels welcher der Schaft ohne Kupplung in einer Solltiefe im Knochen positionierbar ist, eine Vorfixiereinrichtung, mittels welcher am in der Solltiefe positionierten Schaft die Solllage des Lagerkörpers relativ zur Kupplung vorfixierbar ist, und eine Endfixiereinrichtung vorgesehen sind, mittels welcher bei vom Schaft abgenommener Kupplung die vorfixierte Solllage endfixierbar ist.

Erfindungsgemäß kann sowohl auf Probierprothesen als auch auf eine Montagevorrichtung zum Kopieren der mittels einer Probierprothese ermittelten Soll-Relativlage verzichtet werden. Die erfindungsgemäße Positioniereinrichtung gestattet es, den Schaft der Prothese in seiner Solllage und insbesondere in seiner Solltiefe im Knochen zu positionieren, d.h. der Schaft kann sofort definitiv befestigt werden.

Des Weiteren erfolgt erfindungsgemäß die Festlegung der Solllage des Lagerkörpers relativ zur Kupplung direkt am bereits in der Solltiefe positionierten Schaft. Sobald die Solllage des Lagerkörpers relativ zur Kupplung am Schaft vorfixiert ist, können der korrekte Sitz der Prothese sowie die Lage bzw. der Verlauf der Bänder überprüft werden, d.h. bereits zu einem frühen Zeitpunkt während der Operation kann ein einwandfreier Operationsverlauf sichergestellt werden.

Die erfindungsgemäße Vorfixierung, durch die bereits eine stabile Verbindung zwischen dem Lagerkörper und der Kupplung hergestellt wird, ermöglicht es, die Endfixierung der Solllage des Lagerkörpers relativ zur Kupplung in einem vom Schaft abgenommenen Zustand und damit außerhalb des Körpers des Patienten durchzuführen. Während bisher die endgültige Befestigung zwischen Lagerkörper und Kupplung im am Schaft angeordneten Zustand erfolgen und hierzu ein Werkzeug gewissermaßen "von hinten" - d.h. von der dem Lagerkörper gegenüberliegenden Seite des Schaftes her und damit durch den Oberarm und durch eine im Schaft ausgebildete Bohrung hindurch - erfolgen musste, wird durch die Erfindung ein derartiger komplizierter, zu einer eigentlich unnötigen Beeinträchtigung des Patienten führender Fixiervorgang vermieden, indem die Endfixierung der vorfixierten Solllage des Lagerkörpers relativ zur Kupplung außerhalb des Körpers des Patienten durchgeführt wird.

Durch die Erfindung wird folglich bei vergleichsweise geringem Materialaufwand und insbesondere ohne die Verwendung einer Probierprothese und einer Kopiervorrichtung die Operationszeit erheblich verkürzt, die Beeinträchtigung des Patienten minimiert sowie eine große Sicherheit beim Erreichen der korrekten Lage des Lagerkörpers relativ zum Schaft gewährleistet.

Bevorzugt ist vorgesehen, dass die Kupplung zur Verbindung mit dem Schaft einen Spannabschnitt umfasst, mit dem durch Einschlagen in eine Kupplungsaufnahme des Schaftes ein fester Spannsitz der Kupplung im Schaft herstellbar ist. Vorzugsweise weist der Spannabschnitt eine elliptische oder ellipsenartige äußere Querschnittsform auf, wobei alternativ auch ein aus einem gleichseitigen Dreieck abgeleiteter Querschnitt vorgesehen sein kann, der einem so genannten Dreibogengleichdick entspricht. Eine derartige Querschnittsform wird beispielsweise im Maschinenbau als Wellenverbindung eingesetzt.

Des Weiteren ist vorzugsweise vorgesehen, dass sich der Spannabschnitt konusartig verjüngt und in eine entsprechend geformte Kupplungsaufnahme des Schaftes einschlagbar ist.

Die erfindungsgemäße Positioniereinrichtung weist vorzugsweise einen am Schaft fixierbaren Lagerabschnitt sowie wenigstens ein am Lagerabschnitt verschwenkbar und verdrehbar gelagertes, in Richtung einer Längsachse relativ zum Lagerabschnitt axial unbewegliches Positionierelement mit einer dem Schaft zugewandten, als Tiefenanschlag dienenden Unterseite auf, die durch Verschwenken und/oder Verdrehen des Positionierelementes relativ zum Lagerabschnitt in alle für den Lagerkörper in Frage kommende Orientierungen relativ zu einer vorbereiteten ebenen Oberseite (Resektionsfläche) des Knochens bringbar ist.

Mit dem am Lagerabschnitt beweglich gelagerten Positionierelement der erfindungsgemäßen Positioniereinrichtung wird beim Einsetzen des Schaftes in den Knochen gewissermaßen der eigentliche Lagerkörper simuliert, so dass der Schaft bereits in diesem frühen Stadium der Operation in seine Solllage und insbesondere in seine Solltiefe gebracht und damit definitiv gesetzt werden kann.

Vorzugsweise ist der Lagerabschnitt der Positioniereinrichtung als Kugellager ausgebildet, wobei der Mittelpunkt des Kugellagers und die Unterseite des Positionierelementes hinsichtlich ihrer axialen Relativlage derart aufeinander abgestimmt sind, dass bei in der Solltiefe positioniertem Schaft und am Schaft fixiertem Lagerabschnitt die Lage des Mittelpunkts des Kugellagers mit der Solllage des Mittelpunkts eines Kugellagerabschnitts der Kupplung übereinstimmt.

Hierdurch werden die geometrischen Verhältnisse einer korrekt eingesetzten Prothese simuliert, wodurch die korrekte Lage des Schaftes im Knochen mit hoher Genauigkeit und auf einfache Weise sichergestellt werden können.

Das Positionierelement ist bevorzugt derart ausgebildet, dass es hinsichtlich der Form und der Größe seiner ebenen Unterseite sowie der insbesondere exzentrischen Lage einer an der Unterseite mündenden Aufnahme zumindest näherungsweise dem Lagerkörper entspricht.

Bevorzugt ist das Positionierelement in Form einer Ringscheibe vorgesehen.

Das erfindungsgemäße Operationssystem umfasst bevorzugt eine Mehrzahl von Positionierelementen, die sich hinsichtlich der Form und der Größe ihrer ebenen Unterseiten sowie der insbesondere exzentrischen Lage einer an der Unterseite mündenden Aufnahme voneinander unterscheiden.

Der Operateur kann aus diesem Satz von Positionierelementen dasjenige Exemplar auswählen, welches dem für den jeweiligen Patienten benötigten Lagerkörper am nächsten kommt. Des Weiteren wird vorgeschlagen, dass das Positionierelement wenigstens eine Abdrückschraube aufweist, die zum Abdrücken des Positionierelementes von der Oberseite des Knochens aus einer Neutralstellung in eine von der Unterseite des Positionierelementes vorstehende Abdrückstellung bringbar ist.

Insbesondere bei einzuzementierenden Schäften wird hierdurch das Lösen des Positionierelementes vom Knochen erheblich erleichtert.

Des Weiteren ist bevorzugt vorgesehen, dass der Lagerabschnitt der Positioniereinrichtung einen in eine Kupplungsaufnahme des Schaftes einführbaren Kupplungsabschnitt aufweist, der zur Ausrichtung der Positioniereinrichtung relativ zum Schaft eine an die von einer Kreisform abweichende innere Querschnittsform der Kupplungsaufnahme angepasste Außenform aufweist.

Hierdurch wird die korrekte Winkelausrichtung des Lagerabschnitts, die der späteren Soll-Winkelausrichtung der Kupplung der Prothese entspricht, sichergestellt.

Vorzugsweise ist ein Übergang zwischen dem Lagerabschnitt und dem Kupplungsabschnitt der Positioniereinrichtung als Auflage- und Abdichtfläche ausgebildet, die bei am Schaft fixiertem Lagerabschnitt an dem Schaft anliegt und das Schaftinnere gegenüber der Umgebung abdichtet.

Bei Schäften, die durch Einzementieren im Knochen fixiert werden, wird hierdurch das Austreten von Zement sicher verhindert.

Die Fixierung des Lagerabschnitts der Positioniereinrichtung am Schaft erfolgt vorzugsweise mittels einer Spanneinrichtung, wobei die Spanneinrichtung mit einem vorhandenen, in einer Kupplungsaufnahme des Schaftes ausgebildeten Innengewinde zusammenwirkt. Hierdurch kann ein ohnehin vorhandenes Innengewinde des Schaftes in vorteilhafter Weise genutzt werden. Die Spanneinrichtung ist vorzugsweise in Form einer Spannschraube vorgesehen.

Des Weiteren ist bevorzugt vorgesehen, dass der Lagerabschnitt der Positioniereinrichtung zum Aufbringen des Positionierelementes mit einem zylindrischen Oberflächenbereich versehen ist, der bevorzugt durch Anschleifen ausgebildet wird. Hierdurch wird das Aufsetzen des Positionierelementes auf einen kugelförmigen Lagerabschnitt ermöglicht.

Vorzugsweise weist die Mittelachse des zylindrischen Oberflächenbereiches gegenüber einer Längsachse des Lagerabschnitts eine Neigung auf, die außerhalb eines Bereiches von Neigungen liegt, die das Positionierelement während der Schaftpositionierung einnehmen kann. Ein versehentliches Lösen des Positionierelementes vom Lagerabschnitt wird auf diese Weise sicher vermieden.

Ferner umfasst die Vorfixiereinrichtung vorzugsweise ein Stützorgan, das in einer Kupplungsaufnahme des Schaftes axial fixierbar ist und im fixierten Zustand als Endanschlag für einen in der Kupplungsaufnahme durch Einschlagen befestigbaren Spannabschnitt der Kupplung dient, wobei bevorzugt das Stützorgan derart auf die Abmessungen des Schaftes und des Spannabschnitts abgestimmt ist, dass das Stützorgan den Spannabschnitt vor Erreichen einer für die Befestigung erforderlichen Solltiefe in einer ein leichtes Abnehmen der Kupplung ermöglichenden Vorfixiertiefe abfängt.

Das Stützorgan ist vorzugsweise durch Einschrauben in den Schaft unter Ausnutzung eines vorhandenen, in der Kupplungsaufnahme des Schaftes ausgebildeten Innengewindes axial fixierbar.

Ferner umfasst die Vorfixiereinrichtung vorzugsweise ein Ausgleichsorgan, das zum Kompensieren der Differenz zwischen der Solltiefe und der Vorfixiertiefe zwischen den Knochen und den Lagerkörper bringbar und derart ausgebildet ist, dass eine Ausrichtung der Unterseite des Lagerkörpers parallel zur Oberseite des Knochens sichergestellt ist.

Da während des Vorfixierens die Eindringtiefe des Spannabschnitts der Kupplung durch das in die Kupplungsaufnahme des Schaftes eingebrachte Stützorgan begrenzt ist, wird diese an der zur endgültigen Befestigung des Spannabschnitts am Schaft erforderlichen Endtiefe fehlende Differenz während des Vorfixierens durch das Ausgleichsorgan kompensiert.

Vorzugsweise ist das Ausgleichsorgan in Form einer Federscheibe vorgesehen, die bevorzugt einstückig ausgebildet ist und/ oder vorzugsweise mehrere von einer Basisplatte abstehende Federzungen umfasst.

Des Weiteren ist bevorzugt vorgesehen, dass die Vorfixiereinrichtung ein insbesondere konusförmiges Aufspreizelement umfasst, das in einen aufspreizbaren Kugellagerabschnitt der Kupplung einbringbar und durch Einschlagen der Kupplung in den Schaft bei auf deren Kugellagerabschnitt angeordnetem Lagerkörper mittels des im Schaft axial fixierten Stützorgans zum vorfixierenden Aufspreizen des Kugellagerabschnitts in diesen eintreibbar ist.

Damit dient das Stützorgan nicht lediglich als Tiefenanschlag, der während des Vorfixierens ein zu tiefes Eindringen des Spannabschnitts der Kupplung in die Kupplungsaufnahme des Schaftes verhindert, sondern sorgt außerdem bei dem zur Vorfixierung dienenden Einschlagen der Kupplung in den Schaft für das vorfixierende Aufspreizen des Kugellagerabschnitts der Kupplung.

Die erfindungsgemäße Endfixiereinrichtung umfasst vorzugsweise eine Einspannvorrichtung zum Einspannen des an der Kupplung vorfixierten Lagerkörpers zwischen einem Halteorgan und einem Auflageorgan sowie ein Vortriebswerkzeug, mit dem bei eingespanntem Lagerkörper ein den Lagerkörper vorfixierendes Aufspreizelement zum Endfixieren des Lagerkörpers in eine Endfixierstellung treibbar ist. Das Halteorgan ist vorzugsweise axial verstellbar, während das Auflageorgan bevorzug axial fest angeordnet ist.

Das Vortriebswerkzeug ist vorzugsweise zum Eintreiben des Aufspreizelementes unter Ausnutzung eines vorhandenen, in einem Spannabschnitt der Kupplung ausgebildeten Innengewindes in den Spannabschnitt einschraubbar, wobei bevorzugt die Endfixierstellung des Aufspreizelementes durch ein bestimmtes Einschraubdrehmoment des Vortriebswerkzeugs vorgegeben ist.

Des Weiteren wird vorgeschlagen, dass das Auflageorgan eine Auflagefläche für die Unterseite des Lagerkörpers und einen durchgehenden Aufnahmekanal für einen Spannabschnitt der Kupplung aufweist, über den zum einen der Spannabschnitt für das Vortriebswerkzeug zugänglich ist und der zum anderen aufgrund seiner an den von einer Kreisform abweichenden Außenquerschnitt des Spannabschnitts angepassten Innenquerschnittsform als Verdrehsicherung für die Kupplung ausgebildet ist.

Vorzugsweise ist die Auflagefläche des Auflageorgans gegenüber einer Längsachse der Endfixiereinrichtung entsprechend der Neigung des Lagerkörpers relativ zu einer Längsachse der Kupplung geneigt.

Die Endfixierung umfasst bevorzugt zwei durch mehrere Verbindungssäulen in einem festen axialen Abstand gehaltene Grundplatten, wobei das Halteorgan an der einen Grundplatte und das Auflageorgan an der anderen Grundplatte abgestützt ist.

Vorzugsweise umfasst das erfindungsgemäße Operationssystem ferner ein Schlagwerkzeug, mit dem auf den auf dem Kugellagerabschnitt der Kupplung angeordneten Lagerkörper zum Einschlagen der Kupplung in den Schaft Schlagimpulse aufbringbar sind, wobei die Größe eines jeweils aufzubringenden Schlagimpulses am Schlagwerkzeug voreinstellbar und insbesondere veränderbar ist.

Bevorzugt wird das Schlagwerkzeug sowohl zum vorfixierenden Einschlagen der Kupplung in den Schaft als auch zum Herstellen des endgültigen Spannsitzes verwendet.

Weitere bevorzugte Ausführungsformen der Erfindung sind in den Unteransprüchen, der Beschreibung sowie der Zeichnung angegeben.

Die Erfindung wird im Folgenden beispielhaft unter Bezugnahme auf die Zeichnung beschrieben. Es zeigen:
- Fig. 1: schematisch einen in einem Oberarmknochen implantierten Schaft einer Schultergelenkprothese,
- Fig. 2: schematisch ein künstliches Schultergelenk mit einem Gelenkkopf, der einen zu dem in Fig. 1 gezeigten Schaft passenden konischen Spannabschnitt aufweist,
- Fig. 3: schematisch eine Kupplung mit einem Kugellagerabschnitt und einem Spannabschnitt, der durch Einschlagen in den in Fig. 1 dargestellten Schaft festsetzbar ist,
- Fig. 4: schematisch eine Kupplung mit einem Kugellagerabschnitt und einem in den in Fig. 1 dargestellten Schaft durch Einschlagen festsetzbaren Spannabschnitt, wobei auf dem Kugellagerabschnitt ein Gelenkkopf angebracht ist,
- Fig. 5: schematisch in einer Übersichtsdarstellung eine Schultergelenkprothese mit einigen Bestandteilen eines erfindungsgemäßen Operationssystems,
- Fig. 6 - 11: Bestandteile einer Positioniereinrichtung eines erfindungsgemäßen Operationssystems,
- Fig. 12 - 14: Bestandteile einer Vorfixiereinrichtung eines erfindungsgemäßen Operationssystems,
- Fig. 15 - 16: ein Schlagwerkzeug eines erfindungsgemäßen Operationssystems, und
- Fig. 17 - 18: eine Endfixiereinrichtung eines erfindungsgemäßen Operationssystems.

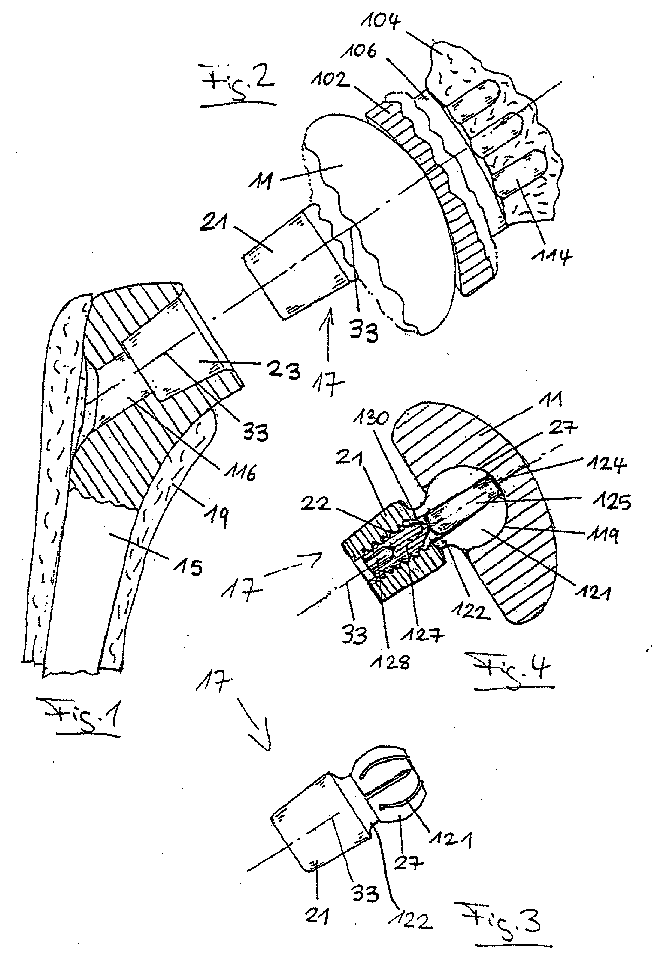

Die Fig. 1 - 4 geben einen Überblick über eine Gelenkprothese in Form einer Schultergelenkprothese, wie sie mit Hilfe des erfindungsgemäßen Operationssystems implantiert werden kann. Bei den mittels des erfindungsgemäßen Operationssystems implantierbaren Gelenkprothesen handelt es sich insbesondere um bereits existierende Prothesen, d.h. der das erfindungsgemäße Operationssystem verwendende Operateur kann weiterhin mit den vorhandenen Prothesen arbeiten. Bei neuen Prothesen kann eine Bohrung 116 in Fig. 1 auch als Sackloch ausgeführt werden, welches gegen lateral geschlossen ist.

Gemäß Fig. 1 und 2 ist ein Prothesenschaft 15 in einem Oberarmknochen 19 implantiert, wobei der Schaft 15 direkt in einem vorbereiteten Knochenbett verankert ist. Alternativ kann es sich bei dem Schaft auch um einen mit Knochenzement im Knochen verankerten Schaft handeln.

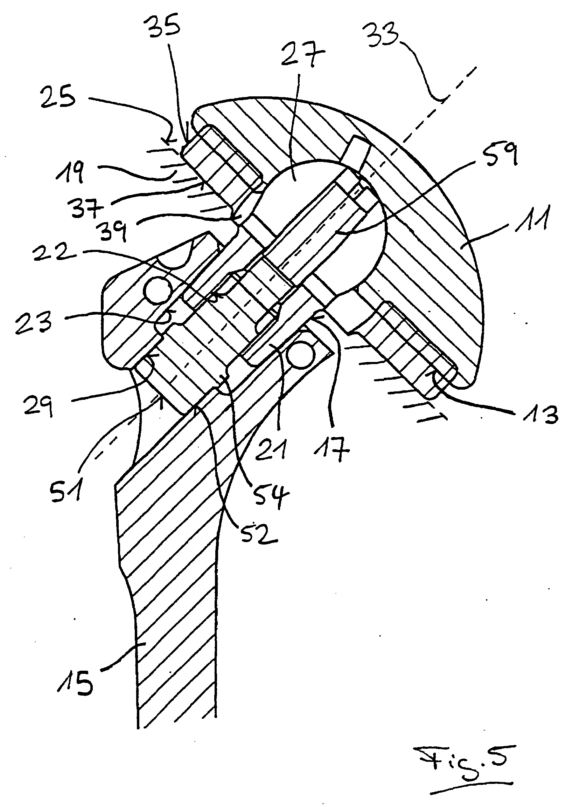

In Richtung einer Längsachse 33 für das eigentliche Schultergelenk ist eine Bohrung 116 im Schaft 15 ausgebildet, die sich zu einer als Gegenform dienenden Kupplungsaufnahme 23 des Schaftes 15 für einen als Spannabschnitt dienenden konischen Körper 21 erweitert. Wie nachstehend näher anhand der Fig. 3 und 4 erläutert wird, ist der Spannabschnitt 21 Bestandteil einer Kupplung 17, die zum Verbinden des Schaftes 15 mit einem als Lagerkopf oder Gelenkkopf ausgebildeten Lagerkörper 11 der Prothese dient.

Das eigentliche Gelenk wird von dem an der Kupplung 17 angebrachten Gelenkkopf 11 sowie einer den anderen Lagerkörper darstellenden Gelenkschale 102 gebildet, die mit einer im Schulterknochen 104 verankerten Plattform 106 starr verbunden ist. Zur Verankerung der Plattform 106 sind parallel zueinander verlaufende Zapfen 114 an der Plattform 106 angebracht, die beispielsweise mit Knochenzement oder durch einen Presssitz in vorbereiteten Bohrungen des Schulterknochens 104 verankert sind.

Der Spannabschnitt 21 sowie entsprechend die als Gegenform für den Spannabschnitt 21 dienende Kupplungsaufnahme 23 weisen jeweils eine elliptisch ausgebildete Querschnittsform auf. Alternativ kann die Querschnittsform auch beispielsweise einem Dreibogengleichdick oder eine andere von einer Kreisform abweichende Form aufweisen.

Die Einzelheiten und Vorteile einer derartigen Verbindung, die einen Spannsitz oder selbsthemmenden Sitz hoher Stabilität und hoher Ausrichtungsgenauigkeit des Spannabschnitts 21 und damit der Kupplung 17 im Schaft 15 ermöglicht, sind nicht Gegenstand der Erfindung und ausführlich in der bereits eingangs erwähnten, noch nicht veröffentlichten europäischen Patentanmeldung EP 0 1 811 120.3 beschrieben.

Mögliche Ausführungsformen für die Kupplung 17 der Gelenkprothese sind in Fig. 3 und Fig. 4 gezeigt. An den Spannabschnitt 21 schließt sich über einen Hals 122 ein als Kugellager für den Gelenkkopf 11 dienender Kugellagerabschnitt 27 an, der eine durchgehende Innenbohrung aufweist und mit Schlitzen 121 versehen ist, durch welche der Kugellagerabschnitt 27 in eine Mehrzahl von Biegeelementen aufgeteilt ist. Um den Kugellagerabschnitt 27 zum Festsetzen des Gelenkkopfes 11 aufzuspreizen, wird in die konisch ausgebildete Innenbohrung 125 des Kugellagerabschnitts 27 ein konisch ausgebildeter Druckkörper 125 über den Spannabschnitt 21 eingeschoben und mittels einer Schraube 127 vorwärts getrieben, wodurch die mittels der Längsschlitze 121 getrennten Lamellen des Kugellagerabschnitts 27 auseinander gespreizt werden. Die Schraube 127 ist als Madenschraube ausgebildet und mit einem Innensechskant 128 versehen. Mit einer Nase 130 treibt die Schraube 127 den Druckkörper 125 weiter in den Kugellagerabschnitt 27 hinein.

Der Druckkörper 125 und die Madenschraube 127, wie sie in Fig. 4 dargestellt sind, sind nicht Bestandteil des nachfolgend erläuterten erfindungsgemäßen Operationssystems, sondern die Fig. 3 und 4 stellen bekannte Lösungen zum Festsetzen eines Gelenkkopfes 11 auf einem Kugellagerabschnitt 27 der Kupplung 17 durch Aufspreizen des Kugellagerabschnitts 27 dar. Das festsetzende Aufspreizen des Kugellagerabschnitts 27 erfolgt bei der Lösung gemäß Fig. 4 mit Hilfe eines Werkzeugs, das bei in der Kupplungsaufnahme 23 befestigtem, axial fixiertem Spannabschnitt 21 von hinten durch den Oberarm und die im Schaft 15 ausgebildete Bohrung 116 hindurch in den Spannabschnitt 21 eingeführt wird, um die Schraube 127 zum Vortreiben des Druckkörpers 125 zu betätigen.

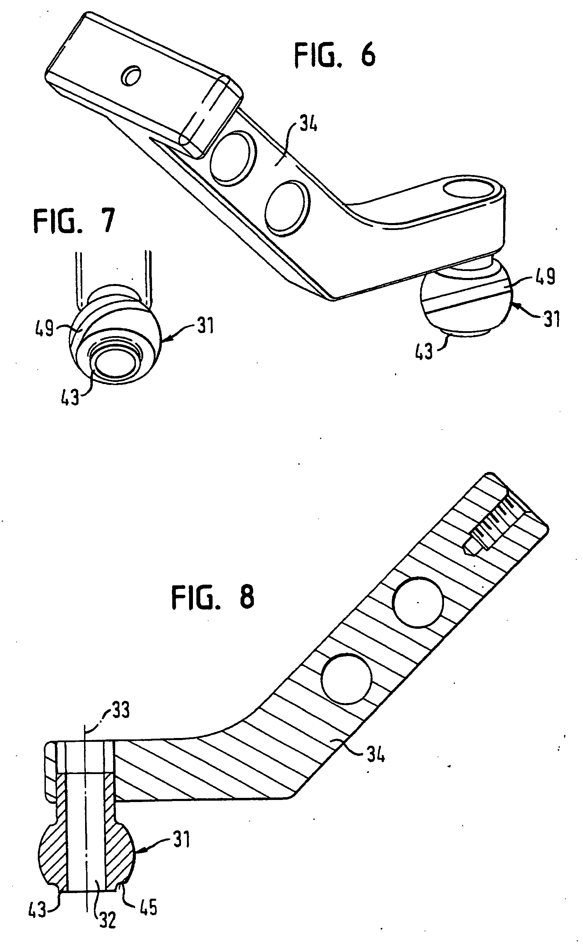

Während die einzelnen Bestandteile des erfindungsgemäßen Operationssystems nachstehend anhand der Fig. 6 - 18 erläutert werden, zeigt Fig. 5 in einer die Zusammenhänge und insbesondere die geometrischen Verhältnisse aufzeigenden Übersichtsdarstellung einen Zustand, der beim tatsächlichen Einsetzen der Prothese während der Operation so nicht eintritt, wie aus den nachstehenden Ausführungen klar wird.

Eine in den Fig. 6 - 10 dargestellte Vorfixiereinrichtung des erfindungsgemäßen Operationssystems umfasst ein Setzwerkzeug mit einem Haltegriff 34, an den ein als Kugellager ausgebildeter Lagerabschnitt 31 befestigt ist, der hinsichtlich Form und Größe zumindest näherungsweise dem Kugellagerabschnitt 27 der für die jeweilige Operation zu verwendenden Kupplung 17 der Prothese (vgl. Fig. 1 - 4) entspricht. Der kugelförmige Lagerabschnitt 31 geht in einen Kupplungsabschnitt 43 über, der in die Kupplungsaufnahme 23 des Schaftes 15 eingeführt werden kann. Der Übergang zwischen dem Lagerabschnitt 31 und dem Kupplungsabschnitt 43 ist derart auf den jeweiligen Schaft 15 abgestimmt, dass er als Auflage- und Abdichtfläche ausgebildet ist, die am Schaft 15 anliegt und im Fall eines einzuzementierenden Schaftes 15 ein Austreten von Zement aus der für den Schaft 15 vorbereiteten Kavität verhindert, wenn während des Vorfixiervorgangs das Setzwerkzeug mit seinem Lagerabschnitt 31 am Schaft 15 fixiert ist.

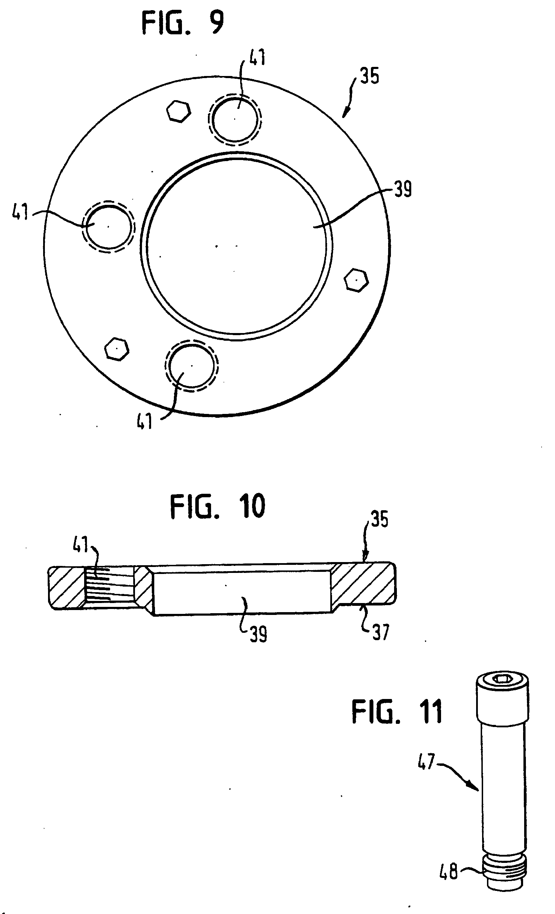

Zu der Positioniereinrichtung gehört ferner ein Satz von jeweils als Ringscheibe ausgebildeten Positionierelementen 35 (vgl. Fig. 9 und 10). Die Ringscheiben 35 weisen jeweils eine von einer Kreisform abweichende äußere Umfangsform auf und sind mit einer exzentrisch angeordneten Aufnahmeöffnung 39 versehen. Ferner sind in jeder Ringscheibe 35 drei jeweils mit einem Innengewinde versehene Durchgänge 41 ausgebildet, in die nicht dargestellte Abdrückschrauben geschraubt werden können, deren Zweck an anderer Stelle näher erläutert wird.

Die Ringscheiben 35 unterscheiden sich voneinander hinsichtlich Form und Größe sowie hinsichtlich der exzentrischen Lage der Aufnahme 39. Dabei ist der Satz von Ringscheiben 35 derart ausgebildet, dass zumindest näherungsweise alle in Frage kommenden Gelenkköpfe 11 (vgl. Fig. 4 und 5) abgedeckt werden, d.h. dass zumindest eine der Ringscheiben 35 wenigstens näherungsweise zu dem in der jeweiligen Operation benötigten Gelenkkopf 11 derart passt, dass die Unterseite 13 des Gelenkkopfes 11 und die Unterseite 37 der Ringscheibe 35 hinsichtlich des äußeren Umrisses und der Lage der exzentrischen Aufnahme 39 miteinander ausgerichtet sind. Hinsichtlich der Unterseite wird folglich der einzusetzende Gelenkkopf 11 durch die passende Ringscheibe 35 simuliert.

Die Aufnahme 39 der Ringscheibe 35 dient dazu, die Ringscheibe 35 auf den kugelförmigen Lagerabschnitt 31 des Setzwerkzeugs aufzusetzen (vgl. Fig. 6 - 8). Hierzu ist der Lagerabschnitt 31 mit einem angeschliffenen zylindrischen Oberflächenbereich versehen 49, dessen Mittelachse zur Längsachse 33 derart geneigt ist, dass diese Neigung außerhalb eines Bereiches von Neigungen liegt, welche die Ringscheibe 35 im aufgesetzten Zustand durch Verschwenken während der eigentlichen Schaftpositionierung einnehmen kann. Der zylindrische Oberflächenbereich 49 ermöglicht also das Aufsetzen der Ringscheibe 35 bei einer Neigung relativ zur Längsachse 33, welche die Ringscheibe 35 während der anschließenden Schaftpositionierung nicht mehr einnimmt.

Im auf den kugelförmigen Lagerabschnitt 31 aufgesetzten Zustand kann die Ringscheibe 35 relativ zum Lagerabschnitt 31 verschwenkt und verdreht werden, wobei die Lage der Ringscheibe 35 relativ zum Lagerabschnitt 31 in Richtung der Längsachse 33 fest ist, d.h. in dieser axialen Richtung die Ringscheibe 35 relativ zum Lagerabschnitt 31 unbeweglich ist, so dass die Unterseite 37 der Ringscheibe 35 während der Schaftpositionierung als ein sicherer und stabiler Tiefenanschlag zur Gewährleistung der korrekten Solltiefe des Schaftes 15 im Knochen 19 dient.

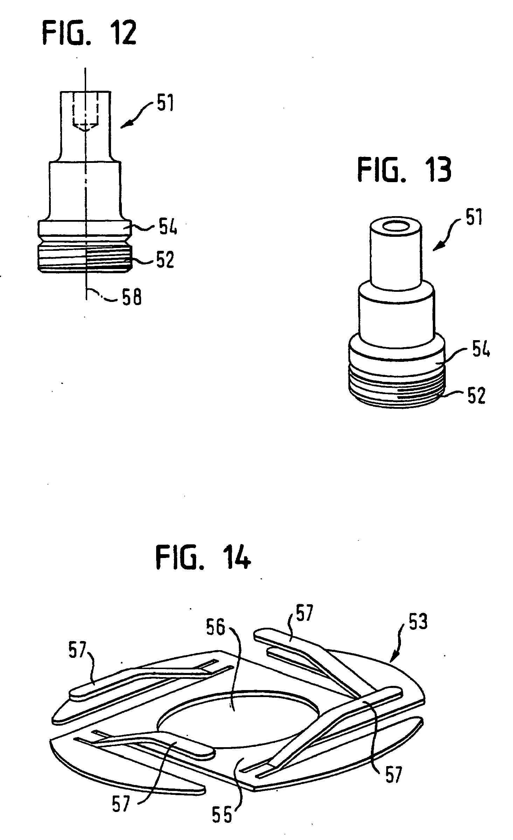

Fig. 11 zeigt eine als Spanneinrichtung dienende Spannschraube 47, mit welcher das Setzwerkzeug (vgl. Fig. 6 - 8) mit dem die lose, d.h. verschwenkbar und verdrehbar, jedoch unverlierbar gehaltene Ringscheibe 35 tragenden Lagerabschnitt 31 am Schaft 15 fixiert werden kann. Hierzu wird die Spannschraube 47 in den Durchgang 32 des Lagerabschnitts 31 eingeführt und über ein am freien Ende der Spannschraube 47 ausgebildetes Außengewinde 48 mit dem in der Kupplungsaufnahme 23 des Schaftes 15 ausgebildeten Innengewinde 29 (vgl. Fig. 5) verschraubt. Auf diese Weise wird mit der erfindungsgemäßen Positioniereinrichtung ein ohnehin vorhandenes Innengewinde 29 des Schaftes 15 ausgenutzt.

Damit der Lagerabschnitt 31 des Setzwerkzeugs korrekt relativ zum Schaft 15 ausgerichtet werden kann, ist die Außenform des in die Kupplungsaufnahme 23 des Schaftes 15 einzuführenden Kupplungsabschnitts 43 des Lagerabschnitts 31 an die von einer Kreisform abweichende, insbesondere elliptische innere Querschnittsform der Kupplungsaufnahme 23 angepasst.

Der kugelförmige Lagerabschnitt 31 des Setzwerkzeugs und die Ringscheibe 35 sind hinsichtlich ihrer axialen Relativlage derart aufeinander abgestimmt, dass die Lage des Mittelpunkts des als Kugellager ausgebildeten Lagerabschnitts 31 mit der Solllage des Mittelpunkts des Kugellagerabschnitts 27 der Kupplung 17 übereinstimmt. Im am Schaft 15 mittels der Spannschraube 47 fixierten Zustand wird durch den kugelförmigen Lagerabschnitt 31 des Setzwerkzeugs folglich der Kugellagerabschnitt 27 der später am Schaft 15 zu befestigenden Kupplung 17 simuliert.

Somit kann bei am Schaft 15 fixierter Positioniereinrichtung die Solllage und insbesondere die Solltiefe des Schaftes 15 im Knochen 19 dadurch eindeutig festgelegt werden, dass sich die Ringscheibe 35, die verschwenkbar und verdrehbar, jedoch axial unbeweglich am Lagerabschnitt 31 gelagert ist, mit ihrer Unterseite 37 an der vorbereiteten Oberseite 25 (Resektionsfläche) des Knochens 19 ausrichtet und diejenige Orientierung einnimmt, die der Sollorientierung des der Unterseite 13 des anschließend mit dem Schaft 15 zu kuppelnden Gelenkkopfes 11 entspricht.

Die Unterseite 37 der Ringscheibe 35 liegt somit am Ende der korrekten Schaftpositionierung genau in derjenigen Ebene, in welcher die Unterseite 13 des Gelenkkopfes 11 später liegen muss.

Die Fig. 12 - 14 zeigen Bestandteile einer Vorfixiereinrichtung des erfindungsgemäßen Operationssystems, und zwar ein Stützorgan 51 (Fig. 12 und 13) sowie eine als Ausgleichsorgan dienende Federscheibe 53 (Fig. 14).

Das an seinem einen axialen Ende mit einem Außengewinde 52 versehene Stützorgan 51 ist in die Kupplungsaufnahme 23 des Schaftes 15 einführbar und in der Kupplungsaufnahme 23 dadurch axial fixierbar, dass es in die mit dem Innengewinde 29 versehene Schaftbohrung eingeschraubt wird, bis eine Ringschulter 54 des Stützorgans 51 an dem Übergang zwischen der Schaftbohrung und der eine größere.Weite aufweisenden Kupplungsaufnahme 23 aufliegt, wie es in Fig. 5 dargestellt ist.

Das dem Außengewindeabschnitt 52 gegenüberliegende axiale Ende des Stützorgans 51 ist derart schlank ausgeführt, dass es in den Spannabschnitt 21 der Kupplung 17 eingeführt werden kann.

Die Federscheibe 53 (vgl. Fig. 14) ist einstückig ausgebildet und umfasst eine Basisplatte 55, in der eine zentrale Kreisöffnung 56 ausgebildet ist und von der in diesem Ausführungsbeispiel vier Federzungen 57 abstehen.

Die Federscheibe 53 dient dazu, während des an anderer Stelle näher erläuterten Vorfixiervorgangs eine parallele Ausrichtung zwischen der Unterseite 13 des Gelenkkopfes 11 einerseits und der Oberseite 25 des Knochens 19 andererseits sicherzustellen. Während des Vorfixiervorgangs befindet sich die Federscheibe 53 folglich an der Stelle zwischen dem Gelenkkopf 11 und dem Knochen 19, an der in Fig. 5 zur Erläuterung der geometrischen Verhältnisse die Ringscheibe 35 dargestellt ist. Insofern entspricht Fig. 5 nicht exakt einer Situation, wie sie sich während eines Vorfixiervorgangs ergibt.

Die Vorfixiereinrichtung des erfindungsgemäßen Operationssystems umfasst des Weiteren ein lediglich in Fig. 5 dargestelltes Aufspreizelement 59, das in den aufspreizbaren Kugellagerabschnitt 27 der Kupplung 17 eingeführt werden kann. Die axiale Länge des Aufspreizelementes 59 und des Stützorgans 51 sind derart aufeinander sowie auf den Spannabschnitt 21 der Kupplung 17 und die Kupplungsaufnahme 23 des Schaftes 15 abgestimmt, dass das in den Spannabschnitt 21 eingeführte schlanke Ende des Stützorgans 51 das Aufspreizelement 59 weiter in den Kugellagerabschnitt 27 hineintreiben kann, um den Kugellagerabschnitt 27 zum Vorfixieren der korrekten Lage des Gelenkkopfes 11 relativ zum Kugellagerabschnitt 27 aufzuspreizen, wenn der Gelenkkopf 11 mit einem zumindest näherungsweise parallel zur Längsachse 33 gerichteten Schlagimpuls beaufschlagt wird. Dabei bildet die Ringschulter 54 auf ihrer Oberseite einen Tiefenanschlag für die Kupplung, der zwar eine Winkelausrichtung des Spannabschnitts 21 in der Kupplungsaufnahme aber "kein Spannen" in der Kupplungsaufnahme 23 sicherstellt.

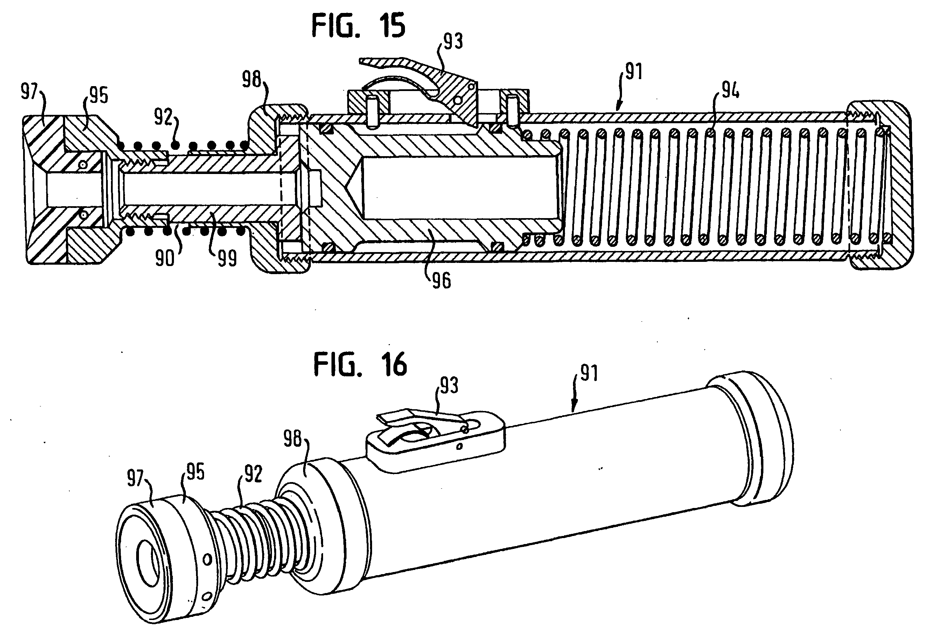

Um zum Vorfixieren der Solllage des Gelenkkopfes 11 relativ zum Kugellagerabschnitt 27 der Kupplung 17 einen Schlagimpuls von angemessener Größe auf den Gelenkkopf 11 aufzubringen, wird ein Schlagwerkzeug 91 des erfindungsgemäßen Operationssystems verwendet, von dem ein mögliches Ausführungsbeispiel in Fig. 15 und 16 dargestellt ist.

In einem am hinteren Ende von einem Deckel verschlossenen Rohr des Schlagwerkzeugs 91 ist eine Impulsfeder 94 angeordnet, die durch Zurückschieben eines ebenfalls im Rohr verschieblich angeordneten Schlaghammers 96 zusammengedrückt werden kann, wobei sich die Impulsfeder 94 am Deckel abstützt. Hierdurch wird das Schlagwerkzeug 91 gespannt.

Im gespannten Zustand hintergreift eine radial nach innen vorstehende Nase eines Auslösers 93 den in Fig. 15 linken Radialvorsprung des Schlaghammers 96, wodurch der gespannte Zustand des Schlagwerkzeugs 91 aufrechterhalten wird.

Auch im gespannten Zustand befindet sich ein an einem Adapter 95 angebrachter, aus Kunststoff hergestellter Schutzkörper 97 in der in Fig. 15 dargestellten axialen Entfernung von einer rohrfesten Führung 98. Hierfür sorgt eine Vorspannfeder 92, welche die Einheit aus Schutzkörper 97 und Adapter 95 in die dargestellte Ausgangsstellung vorspannt.

Mit dem Adapter 95 axial fest gekoppelt ist ein Stößel 99, der von der Führung 98 axial beweglich geführt ist und dazu dient, den mittels des nach dem Auslösen zurückfedernden Schlaghammers 96 erzeugten Impuls über den Adapter 95 auf den Schutzkörper 97 zu übertragen. Wenn das Schlagwerkzeug 91 mit am Gelenkkopf 11 anliegendem Schutzkörper 97 gegen den Gelenkkopf 11 gedrückt wird, kann gegen die Kraft der Vorspannfeder 92 die Einheit aus Schutzkörper 97 und Adapter 95 so weit in Richtung der Führung 98 bewegt werden, wie es der zwischen den einander zugewandten Stirnseiten des Adapters 95 und der Führung 98 in der Ausgangsstellung vorhandene axiale Zwischenraum 90 zulässt.

Das Zurückschieben des Schlaghammers 96 zum Spannen oder "Laden" des Schlagwerkzeugs 91 erfolgt durch eine nicht dargestellte Ladehilfe, die durch im Schutzkörper 97, im Adapter 95 und im Stößel 99 ausgebildete Durchgänge hindurchführbar ist und auf diese Weise den Schlaghammer 96 gegen die Kraft der Impulsfeder 94 beaufschlagen kann.

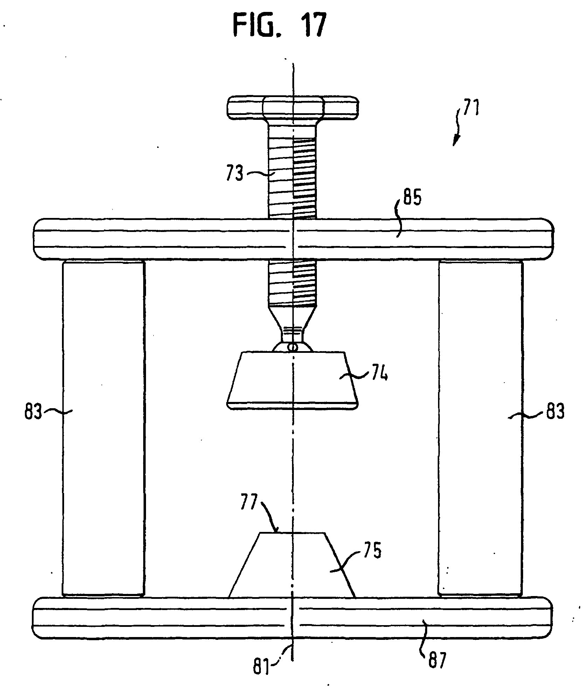

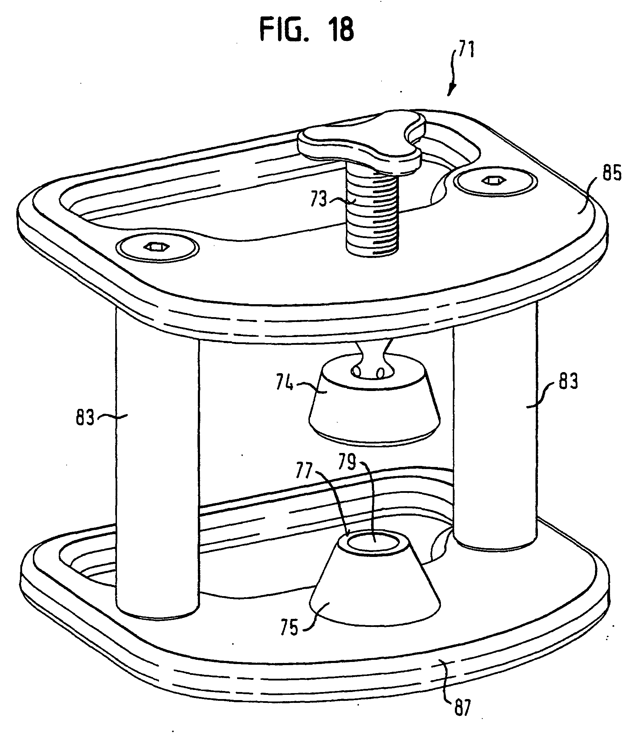

Eine Endfixiereinrichtung des erfindungsgemäßen Operationssystems umfasst eine in Fig. 17 und 18 dargestellte Einspannvorrichtung 71, die zwei Grundplatten 85, 87 aufweist, welche durch zwei Verbindungssäulen 83 in einem festen axialen Abstand voneinander gehalten werden.

Die Einspannvorrichtung 71 umfasst ferner ein axial relativ zur oberen Grundplatte 85 verstellbares Halteorgan 73, das an seinem zwischen den Grundplatten 85, 87 befindlichen freien Ende einen verschwenkbar gelagerten, aus Kunststoff hergestellten Kontaktkörper 74 trägt. An der Innenseite der unteren Grundplatte 87 ist ein ebenfalls aus Kunststoff hergestelltes Auflageorgan 75 angeordnet, dessen dem Kontaktkörper 74 zugewandte Stirnseite als Auflagefläche 77 für die Unterseite 13 (vgl. Fig. 5) eines einzuspannenden Gelenkkopfes 11 dient, der somit im eingespannten Zustand zwischen dem entsprechend axial positionierten Kontaktkörper 74 und dem Auflageorgan 75 fest eingespannt ist, ohne die Oberflächen des Gelenkkopfes 11 zu beschädigen.

Das Auflageorgan 75 ist auswechselbar an der unteren Grundplatte 87 angebracht, so dass unterschiedlich ausgebildete Auflageorgane 75 eingesetzt werden können, um die Einspannvorrichtung 71 an die jeweilige Einheit aus Kupplung 17 und am Kugellagerabschnitt 27 der Kupplung 17 endzufixierenden Gelenkkopf 11 anzupassen. Anders als in Fig. 17 und 18 dargestellt, kann dabei die Auflagefläche 77 des Auflageorgans 75 auch gegenüber der Längsachse 81 (vgl. Fig. 17) der Einspannvorrichtung 71 geneigt verlaufen, und zwar z.B. entsprechend der Sollneigung des Gelenkkopfes 11 relativ zur Längsachse 33 der Kupplung 17.

Im Auflageorgan 75 ist ein durchgehender Aufnahmekanal 79 (vgl. Fig. 18) ausgebildet, der mit einer Öffnung in der unteren Grundplatte 87 ausgerichtet ist, so dass der Spannabschnitt 21 der eingespannten Einheit aus Kupplung 17 und Gelenkkopf 11 von unten für ein Vortriebswerkzeug (nicht dargestellt) der erfindungsgemäßen Endfixiereinrichtung zugänglich ist. Das Vortriebswerkzeug umfasst eine an ihrem einen axialen Ende einen Außengewindeabschnitt aufweisende Spannschraube, die mittels eines Drehmomentschlüssels über das Innengewinde 22 des Spannabschnitts 21 (vgl. Fig. 5) in den Spannabschnitt 21 einschraubbar ist, um auf diese Weise das im Kugellagerabschnitt 27 der Kupplung 17 befindliche Aufspreizelement 59 (vgl. Fig. 5) weiter in den Kugellagerabschnitt 27 hineinzutreiben, bis bei Erreichen eines vorgegebenen Drehmoments der Kugellagerabschnitt 27 derart weit aufgespreizt ist, dass der Gelenkkopf 11 am Kugellagerabschnitt 27 und damit an der Kupplung 17 in der gewünschten Solllage endfixiert ist.

Das erfindungsgemäße Operationssystem ist in einem Verfahren zum Einsetzen einer Gelenkprothese, wie sie vorstehend beschrieben wurde, einsetzbar, bei dem zunächst mittels einer Positioniereinrichtung (Fig. 6 - 10) der Schaft 15 ohne Kupplung 17 in einer Solltiefe im Knochen 19 beispielsweise durch Einschlagen oder Einzementieren befestigt wird, anschließend mittels einer Vorfixiereinrichtung (Fig. 12 - 14) am in der Solltiefe positionierten Schaft 15 die Solllage des Lagerkörpers 11 relativ zur Kupplung 17 vorfixiert wird, und schließlich mittels einer Endfixiereinrichtung (Fig. 17 und 18) bei vom Schaft 15 abgenommener Kupplung 17 die vorfixierte Solllage endfixiert wird.

Bei dem Verfahren wird insbesondere vor oder nach dem bevorzugt durch Raspeln erfolgenden Ausbildung der zur Aufnahme des Schaftes 15 dienenden Kavität im Knochen 19 aus einer Mehrzahl von Positionierelementen 35 (Ringscheiben) der Positioniereinrichtung, die sich hinsichtlich der Form und der Größe ihrer ebenen Unterseiten 37 sowie der insbesondere exzentrischen Lage einer an der Unterseite 37 mündenden Aufnahme 39 voneinander unterscheiden, ein passendes Positionierelement 35 (Ringscheibe), das hinsichtlich der Form und der Größe seiner Unterseite 37 sowie der Lage der Aufnahme 39 zumindest näherungsweise dem Lagerkörper 11 (Gelenkkopf) entspricht, anhand eines zuvor zur Vorbereitung des Knochens 19 von diesem abgetrennten, insbesondere kalottenförmigen oder kalottenartig geformten Knochenstücks ausgewählt.

Anschließend wird die ausgewählte Ringscheibe 35 an dem Lagerabschnitt 31 der Positioniereinrichtung verschwenkbar und verdrehbar sowie in Richtung einer Längsachse 33 relativ zum Lagerabschnitt 31 axial unbeweglich angebracht. Hierzu dient - wie vorstehend erwähnt - der zylindrische Anschliff 49 des Lagerabschnitts 31.

Dann wird der die ausgewählte Ringscheibe 35 tragende Lagerabschnitt 31 mittels einer Spanneinrichtung 47 (Spannschraube) der Positioniereinrichtung am Schaft 15 fixiert. Hierbei wirkt die Spannschraube 47 mit dem vorhandenen, in der Kupplungsaufnahme 23 des Schaftes 15 ausgebildeten Innengewinde 29 zusammen.

Danach wird der Schaft 15, der mit dem die ausgewählte Ringscheibe 35 tragenden Lagerabschnitt 31 fest verbunden ist, in die vorbereitete Kavität des Knochens 19 eingeführt und in seine endgültige Solllage, insbesondere in die endgültige Solltiefe, im Knochen 19 gebracht. Diese Solllage wird mit Hilfe der erfindungsgemäßen Positioniereinrichtung dadurch bestimmt, dass sich die verschwenkbar und verdrehbar, axial unbeweglich am Lagerabschnitt 31 gelagerte Ringscheibe 35 mit ihrer Unterseite 37 an der Resektionsfläche 25 des Knochens 19 in einer Orientierung ausrichtet, die der Sollorientierung des anschließend mit dem Schaft 15 zu kuppelnden Gelenkkopfes 11 bzw. dessen Unterseite 13 entspricht.

Beim Ansetzen des Lagerabschnitts 31 an den Schaft 15 wird die korrekte Ausrichtung des Lagerabschnitts 31 relativ zum Schaft 15 durch die Außenform des Kupplungsabschnitts 43 des Lagerabschnitts 31 sichergestellt, die an die von einer Kreisform abweichende, insbesondere elliptische innere Querschnittsform der Kupplungsaufnahme 23 des Schaftes 15 angepasst ist. Insofern simuliert der Kupplungsabschnitt 43 des Lagerabschnitts 31 den Spannabschnitt 21 der Kupplung 17.

Wenn die Solllage des Schaftes 15 im Knochen 19 erreicht ist, wird der Schaft 15 entweder durch Einzementieren oder durch Einschlagen definitiv fixiert. Im Fall des Einzementierens verhindert die am Schaft 15 anliegende Auflage- und Abdichtfläche 45, die im Übergang zwischen Lagerabschnitt 31 und Kupplungsabschnitt 43 (vgl. Fig. 8) ausgebildet ist, ein Austreten des Zementes aus der Kavität des Schaftes 15.

Nach dem Einschlagen bzw. dem Aushärten des Zementes werden der Lagerabschnitt 31 und die Ringscheibe 35 vom Schaft 15 und vom Knochen 19 abgenommen. Die bei einem einzementierten Schaft 15 eventuell aufgrund des Zementes am Knochen 19 haftende Ringscheibe 35 kann dabei von der Oberseite 25 des Knochens 19 dadurch abgedrückt werden, dass die vorstehend erwähnten, in den Schraubendurchgängen 41 sitzenden Abdrückschrauben der Ringscheibe 35 von oben betätigt und hierdurch aus einer Neutralstellung in eine von der Unterseite 37 der Ringscheibe 35 vorstehende Abdrückstellung geschraubt werden.

Anschließend erfolgt am bereits seine Solllage im Knochen 19 einnehmenden Schaft 15 das Vorfixieren der Solllage des Gelenkkopfes 11 relativ zur Kupplung 17, indem zunächst ein Stützorgan 51 der erfindungsgemäßen Vorfixiereinrichtung in der Kupplungsaufnahme 23 des Schaftes 15 axial fixiert wird. Dies erfolgt insbesondere durch Einschrauben des Stützorgans 51 in den Schaft 15 unter Ausnutzung eines vorhandenen, in der Kupplungsaufnahme 23 des Schaftes 15 ausgebildeten Innengewindes 29.

Außerdem wird ein beispielsweise konusförmiges Aufspreizelement 59 der Vorfixiereinrichtung in den aufspreizbaren Kugellagerabschnitt 27 der Kupplung 17 eingebracht. Dabei wird insbesondere das Aufspreizelement 59 mittels eines einen Tiefenanschlag aufweisenden Stößels (nicht dargestellt) in eine vorgegebene Ausgangslage gebracht, in welcher der Kugellagerabschnitt 27 der Kupplung 17 lediglich geringfügig aufgespreizt wird, so dass der Gelenkkopf 11 noch verdreht und verschwenkt werden kann, wenn der Gelenkkopf 11 anschließend auf den leicht aufgespreizten Kugellagerabschnitt 27 aufgesteckt wird. Durch das Eindrücken des Aufspreizelementes 59 zum geringfügigen Aufspreizen des Kugellagerabschnitts 27 der Kupplung 17 ist außerdem sichergestellt, dass das Aufspreizelement 59 nicht versehentlich aus der Kupplung 17 herausfällt.

Anschließend wird die den noch verschwenkbaren und verdrehbaren Lagerkörper 11 tragende Kupplung 17 mit ihrem Spannkonus 21 in die das axial fixierte Stützorgan 51 beinhaltende Kupplungsaufnahme 23 des Schaftes 15 eingeführt. Dabei ist - vgl. Fig. 5 - das Stützorgan 51 derart auf die Abmessungen des Schaftes 15 und des Spannabschnitts 21 abgestimmt, dass der Spannabschnitt 21 beim Einführen in die Kupplungsaufnahme 23 vor Erreichen einer für seine Befestigung im Schaft 15 erforderlichen Solltiefe in einer ein Abnehmen der Kupplung 17 ermöglichenden Vorfixiertiefe vom Stützorgan 51 abgefangen wird.

Vor dem eigentlichen Vorfixiervorgang wird der Gelenkkopf 11 durch Verdrehen relativ zum Kugellagerabschnitt 27 der Kupplung 17 am Knochen 19 ausgerichtet und hierdurch bezüglich des Drehwinkels in seine Solllage relativ zum Knochen 19 gebracht.

Vor dem Einführen des Spannabschnitts 21 der Kupplung 17 in die Kupplungsaufnahme 23 des Schaftes 15 wird ein Ausgleichsorgan 53 (Federscheibe) der Vorfixiereinrichtung zwischen den Knochen 19 und den Gelenkkopf 11 gebracht, um die Differenz zwischen der Solltiefe und der Vorfixiertiefe des Spannabschnitts 21 zu kompensieren. Hierdurch wird die parallele Ausrichtung der Unterseite 13 des Gelenkkopfes 11 parallel zur Resektionsfläche 25 des Knochens 19 sichergestellt.

Abgesehen von der in Fig. 5 zur Erläuterung der geometrischen Verhältnisse dargestellten Ringscheibe 35 zwischen Gelenkkopf 11 und Knochen 19 entspricht die Situation zu diesem Zeitpunkt dem in Fig. 5 gezeigten Zustand. Dabei ist zwischen dem Spannkonus 21 und der Kupplungsaufnahme 23 noch Luft vorhanden, d.h. der Spannkonus 21 kann noch weiter in den Schaft 15 hineingeschlagen werden, bevor er vom Stützorgan 51 durch die obere Seite der Ringschulter 54 abgefangen wird.

Dann wird insbesondere mittels eines Schlagwerkzeugs 91 (vgl. Fig. 15 und 16) ein Schlagimpuls vorgegebener Größe derart auf den Gelenkkopf 11 aufgebracht, dass das zuvor in den Kugellagerabschnitt 27 der Kupplung 17 eingebrachte Aufspreizelement 59 durch das im Schaft 15 axial fixierte Stützorgan 51 (vgl. Fig. 5) weiter in den Kugellagerabschnitt 27 hineingetrieben und dadurch der Kugellagerabschnitt 27 derart weit aufgespreizt wird, dass der Gelenkkopf 11 in seiner Solllage relativ zur Kupplung 17 vorfixiert wird. Durch die zuvor zwischen den Knochen 19 und den Gelenkkopf 11 gebrachte Federscheibe 53 wird hierbei eine Ausrichtung der Unterseite 13 des Gelenkkopfes 11 parallel zur Oberseite 25 des Knochens 19 sichergestellt.

Da der Spannabschnitt 21 rechtzeitig vom Stützorgan 51 in der Vorfixiertiefe abgefangen wird, kann die Kupplung 17 im Anschluss an den Vorfixiervorgang problemlos vom Schaft 15 abgenommen werden. Das Stützorgan 51 kann dann aus dem Schaft 15 durch Herausschrauben entfernt werden.

Zum Endfixieren der nunmehr vorfixierten Solllage des Gelenkkopfes 11 relativ zur Kupplung 17 wird der Gelenkkopf 11 zwischen dem Halteorgan 73 und dem Auflageorgan 75 der erfindungsgemäßen Endfixiereinrichtung eingespannt (vgl. Fig. 17 und 18). Im eingespannten Zustand wird mittels eines Vortriebswerkzeugs das den Gelenkkopf 11 vorfixierende Aufspreizelement 59 weiter in den aufspreizbaren Kugellagerabschnitt 27 der Kupplung 17 hinein in eine vorgegebene Endfixierstellung getrieben. Dabei nutzt das Vortriebswerkzeug insbesondere das vorhandene, im Spannabschnitt 21 der Kupplung 17 ausgebildete Innengewinde 22, indem das Vortriebswerkzeug in den Spannabschnitt 21 eingeschraubt wird und dabei das Aufspreizelement 59 vortreibt.

Während dieses Eintreibens des Aufspreizelementes 59 in den Kugellagerabschnitt 27 der Kupplung 17 wird deren Spannabschnitt 21 mittels des Auflageorgans 75 gegen ein Verdrehen gesichert. Hierzu ist das Auflageorgan 75 mit einem innen zumindest bereichsweise entsprechend der von einer Kreisform abweichenden, insbesondere elliptischen Außenform des Spannabschnitts 21 geformten Aufnahmekanal 79 für den Spannabschnitt 21 versehen.

Anschließend wird bei aus der Kupplungsaufnahme 23 des Schaftes 15 entferntem Stützorgan 51 die den nunmehr endfixierten Gelenkkopf 11 tragende Kupplung 17 mit ihrem Spannkonus 21 in die Kupplungsaufnahme 23 des Schaftes 15 eingeführt. Es wird dann wiederum insbesondere mittels des Schlagwerkzeugs 91 (vgl. Fig. 15 und 16) auf den Gelenkkopf 11 ein Schlagimpuls vorgegebener Größe derart aufgebracht, dass der Spannabschnitt 21 in den endgültigen festen Spannsitz in der Kupplungsaufnahme 23 geschlagen und damit der Gelenkkopf 11 in seine Solllage relativ zum Knochen gebracht wird. Nachdem der Spannkonus 21 nun nicht mehr vom Stützorgan 51 abgefangen werden kann, kann der Spannkonus 21 in die für seine Befestigung im Schaft 15 erforderliche Solltiefe geschlagen werden.

Vorzugsweise ist vorgesehen, dass beim Einführen des Spannabschnitts 21 in den Schaft 15 sowohl zum Vorfixieren als auch zum Herstellen des endgültigen Spannsitzes ein elliptischer Querschnitt des Spannabschnitts 21 derart relativ zur Kupplungsaufnahme 23 des Schaftes 15 in seiner Ebene ausgerichtet wird, dass die große Ellipsenachse in einer Projektion gegen lateral als Senkrechte erscheint.

### Bezugszeichenliste

- 11: Lagerkörper, Gelenkkopf
- 13: Unterseite des Lagerkörpers
- 15: Schaft
- 17: Kupplung
- 19: Knochen
- 21: Spannabschnitt der Kupplung, Spannkonus
- 22: Innengewinde des Spannabschnitts
- 23: Kupplungsaufnahme des Schaftes
- 25: Oberseite des Knochens, Resektionsfläche
- 27: Kugellagerabschnitt der Kupplung
- 29: Innengewinde des Schaftes

- 31: Lagerabschnitt
- 32: Durchgang
- 33: Längsachse
- 34: Haltegriff
- 35: Positionierelement, Ringscheibe
- 37: Unterseite des Positionierelementes
- 39: Aufnahme des Positionierelementes
- 41: Schraubendurchgang des Positionierelementes
- 43: Kupplungsabschnitt
- 45: Auflage- und Abdichtfläche
- 47: Spanneinrichtung, Spannschraube
- 48: Außengewinde der Spanneinrichtung
- 49: zylindrischer Oberflächenbereich

- 51: Stützorgan
- 52: Außengewinde
- 53: Ausgleichsorgan, Federscheibe
- 54: Ringschulter
- 55: Basisplatte
- 56: Öffnung
- 57: Federzunge
- 58: Längsachse des Stützorgans
- 59: Aufspreizelement

- 71: Einspannvorrichtung
- 73: Halteorgan
- 74: Kontaktkörper
- 75: Auflageorgan
- 77: Auflagefläche
- 79: Aufnahmekanal
- 81: Längsachse
- 83: Verbindungssäule
- 85: Grundplatte
- 87: Grundplatte

- 90: Zwischenraum
- 91: Schlagwerkzeug
- 92: Vorspannfeder
- 93: Auslöser
- 94: Impulsfeder
- 95: Adapter
- 96: Schlaghammer
- 97: Schutzkörper
- 98: Führung
- 99: Stößel

- 102: Gelenkschale
- 104: Schulterknochen
- 106: Plattform
- 114: Zapfen
- 116: Bohrung
- 119: Ausnehmung
- 121: Schlitz
- 122: Hals
- 124: konische Bohrung
- 125: Druckkörper
- 127: Schraube
- 128: Innensechskant
- 130: Nase

## Patentansprüche

1. Operationssystem für eine zwei zusammenwirkende Lagerkörper (11, 102), insbesondere einen Gelenkkopf (1,1) und eine Gelenkschale (102), einen Schaft (15) und eine Kupplung (17) zum Verbinden des Schaftes (15) mit einem der Lagerkörper (11) aufweisende Gelenkprothese, insbesondere Schultergelenkprothese,
**gekennzeichnet durch**
- eine Positioniereinrichtung, mittels welcher der Schaft (15) ohne Kupplung (17) in einer Solltiefe im Knochen (19) positionierbar ist,
- eine Vorfixiereinrichtung, mittels welcher am in der Solltiefe positionierten Schaft (15) die Solllage des Lagerkörpers (11) relativ zur Kupplung (17) vorfixierbar ist, und
- eine Endfixiereinrichtung, mittels welcher bei vom Schaft (15) abgenommener Kupplung (17) die vorfixierte Solllage endfixierbar ist.

2. Operationssystem nach Anspruch 1,
**dadurch gekennzeichnet,**
**dass** die Kupplung (17) zur Verbindung mit dem Schaft (15) einen Spannabschnitt (21) umfasst, mit dem durch Einschlagen in eine Kupplungsaufnahme (23) des Schaftes (15) ein fester Spannsitz der Kupplung (17) im Schaft (15) herstellbar ist.

3. Operationssystem nach Anspruch 2,
**dadurch gekennzeichnet,**
**dass** der Spannabschnitt (21) sich konusartig verjüngt und in eine entsprechend geformte Kupplungsaufnahme (23) des Schaftes (15) einschlagbar ist, wobei vorzugsweise der Spannabschnitt (21) einen von einer Kreisform abweichenden, insbesondere elliptischen Außenquerschnitt aufweist.

4. Operationssystem nach einem der vorhergehenden Ansprüche,
**dadurch gekennzeichnet,**
**dass** die Positioniereinrichtung einen am Schaft fixierbaren Lagerabschnitt (31) sowie wenigstens ein am Lagerabschnitt (31) verschwenkbar und verdrehbar gelagertes, in Richtung einer Längsachse (33) relativ zum Lagerabschnitt (31) axial unbewegliches Positionierelement (35) mit einer dem Schaft (15) zugewandten, als Tiefenanschlag dienenden Unterseite (37) aufweist, die durch Verschwenken und/oder Verdrehen des Positionierelementes (35) relativ zum Lagerabschnitt (31) in alle für den Lagerkörper (11) in Frage kommenden Orientierungen relativ zu einer vorbereiteten ebenen Oberseite (25) des Knochens (19) bringbar ist.

5. Operationssystem nach Anspruch 4,
**dadurch gekennzeichnet,**
**dass** der Lagerabschnitt (31) der Positioniereinrichtung als Kugellager ausgebildet ist, wobei der Mittelpunkt des Kugellagers und die Unterseite (37) des Positionierelementes (35) hinsichtlich ihrer axialen Relativlage derart aufeinander abgestimmt sind, dass bei in der Solltiefe positioniertem Schaft (15) und am Schaft (15) fixiertem Lagerabschnitt (31) die Lage des Mittelpunkts des Kugellagers mit der Solllage des Mittelpunkts eines Kugellagerabschnitts (27) der Kupplung (17) übereinstimmt.

6. Operationssystem nach Anspruch 4 oder 5,
**dadurch gekennzeichnet,**
**dass** das Positionierelement (35) hinsichtlich der Form und der Größe seiner ebenen Unterseite (37) sowie der insbesondere exzentrischen Lage einer an der Unterseite (37) mündenden Aufnahme (39) zumindest näherungsweise dem Lagerkörper (11) entspricht.

7. Operationssystem nach einem der Ansprüche 4 bis 6,
**dadurch gekennzeichnet,**
**dass** das Positionierelement (35) in Form einer Ringscheibe vorgesehen ist.

8. Operationssystem nach einem der Ansprüche 4 bis 7,
**dadurch gekennzeichnet,**
**dass** eine Mehrzahl von Positionierelementen (35) vorgesehen ist, die sich hinsichtlich der Form und der Größe ihrer ebenen Unterseiten (37) sowie der insbesondere exzentrischen Lage einer an der Unterseite (37) mündenden Aufnahme (39) voneinander unterscheiden.

9. Operationssystem nach einem der Ansprüche 4 bis 8,
**dadurch gekennzeichnet,**
**dass** das Positionierelement (35) wenigstens eine Abdrückschraube aufweist, die zum Abdrücken des Positionierelementes (35) von der Oberseite (25) des Knochens (19) aus einer Neutralstellung in eine von der Unterseite (37) des Positionierelementes (35) vorstehende Abdrückstellung bringbar ist.

10. Operationssystem nach einem der Ansprüche 4 bis 9,
**dadurch gekennzeichnet,**
**dass** der Lagerabschnitt (31) der Positioniereinrichtung einen in eine Kupplungsaufnahme (23) des Schaftes (15) einführbaren Kupplungsabschnitt (43) aufweist, der zur Ausrichtung der Positioniereinrichtung relativ zum Schaft (15) eine an die von einer Kreisform abweichende innere Querschnittsform der Kupplungsaufnahme (23) angepasste Außenform aufweist.

11. Operationssystem nach einem der Ansprüche 4 bis 10,
**dadurch gekennzeichnet,**
**dass** ein Übergang zwischen dem Lagerabschnitt (31) und dem Kupplungsabschnitt (43) der Positioniereinrichtung als Auflage- und Abdichtfläche (45) ausgebildet ist, die bei am Schaft (15) fixiertem Lagerabschnitt (31) an dem Schaft (15) anliegt und das Schaftinnere gegenüber der Umgebung abdichtet.

12. Operationssystem nach einem der Ansprüche 4 bis 11,
**dadurch gekennzeichnet,**
**dass** der Lagerabschnitt (31) mittels einer Spanneinrichtung (47) der Positioniereinrichtung am Schaft (15) fixierbar ist, wobei die Spanneinrichtung (47) mit einem vorhandenen, in einer Kupplungsaufnahme (23) des Schaftes (15) ausgebildeten Innengewinde (29) zusammenwirkt.

13. Operationssystem nach einem der Ansprüche 4 bis 11,
**dadurch gekennzeichnet,**
**dass** der Lagerabschnitt (31) der Positioniereinrichtung zum Aufbringen des Positionierelementes (35) mit einem zylindrischen Oberflächenbereich (49) versehen ist.

14. Operationssystem nach Anspruch 13,
**dadurch gekennzeichnet,**
**dass** die Mittelachse des zylindrischen Oberflächenbereiches (49) gegenüber einer Längsachse (33) des Lagerabschnitts (31) eine Neigung aufweist, die außerhalb eines Bereiches von Neigungen liegt, die das Positionierelement (35) während der Schaftpositionierung einnehmen kann.

15. Operationssystem nach einem der vorhergehenden Ansprüche,
**dadurch gekennzeichnet,**
**dass** die Vorfixiereinrichtung ein Stützorgan (51) umfasst, das in einer Kupplungsaufnahme (23) des Schaftes (15) axial fixierbar ist und im fixierten Zustand als Endanschlag für einen in der Kupplungsaufnahme (23) durch Einschlagen befestigbaren Spannabschnitt (21) der Kupplung (17) dient, wobei bevorzugt das Stützorgan (51) derart auf die Abmessungen des Schaftes (15) und des Spannabschnitts (21) abgestimmt ist, dass das Stützorgan (51) den Spannabschnitt (21) vor Erreichen einer für die Befestigung erforderlichen Solltiefe in einer ein leichtes Abnehmen der Kupplung (17) ermöglichenden Vorfixiertiefe abfängt.

16. Operationssystem nach Anspruch 15,
**dadurch gekennzeichnet,**
**dass** das Stützorgan (51) durch Einschrauben in den Schaft (15) unter Ausnutzung eines vorhandenen, in der Kupplungsaufnahme (23) des Schaftes (15) ausgebildeten Innengewindes (29) axial fixierbar ist.

17. Operationssystem nach Anspruch 15 oder 16,
**dadurch gekennzeichnet,**
**dass** die Vorfixiereinrichtung ein Ausgleichsorgan (53) umfasst, das zum Kompensieren der Differenz zwischen der Solltiefe und der Vorfixiertiefe zwischen den Knochen (19) und den Lagerkörper (11) bringbar und derart ausgebildet ist, dass eine Ausrichtung der Unterseite (13) des Lagerkörpers (11) parallel zur Oberseite (25) des Knochens (19) sichergestellt ist.

18. Operationssystem nach Anspruch 17,
**dadurch gekennzeichnet,**
**dass** das Ausgleichsorgan (53) in Form einer Federscheibe vorgesehen ist, die vorzugsweise einstückig ausgebildet ist und/oder bevorzugt mehrere von einer Basisplatte (55) abstehende Federzungen (57) umfasst.

19. Operationssystem nach einem der vorhergehenden Ansprüche,
**dadurch gekennzeichnet,**
**dass** die Vorfixiereinrichtung ein insbesondere konusförmiges Aufspreizelement (59) umfasst, das in einen aufspreizbaren Kugellagerabschnitt (27) der Kupplung (17) einbringbar und durch Einschlagen der Kupplung (17) in den Schaft (15) bei auf deren Kugellagerabschnitt (27) angeordnetem Lagerkörper (11) mittels des im Schaft (15) axial fixierten Stützorgans (51) zum vorfixierenden Aufspreizen des Kugellagerabschnitts (27) in diesen eintreibbar ist.

20. Operationssystem nach einem der vorhergehenden Ansprüche,
**dadurch gekennzeichnet,**
**dass** die Endfixiereinrichtung eine Einspannvorrichtung (71) zum Einspannen des an der Kupplung (17) vorfixierten Lagerkörpers (11) zwischen einem bevorzugt axial verstellbaren Halteorgan (73) und einem bevorzugt axial festen Auflageorgan (75) sowie ein Vortriebswerkzeug umfasst, mit dem bei eingespanntem Lagerkörper (11) ein den Lagerkörper (11) vorfixierendes Aufspreizelement (59) zum Endfixieren des Lagerkörpers (11) in eine Endfixierstellung treibbar ist.

21. Operationssystem nach Anspruch 20,
**dadurch gekennzeichnet,**
**dass** das Vortriebswerkzeug zum Eintreiben des Aufspreizelementes (59) unter Ausnutzung eines vorhandenen, in einem Spannabschnitt (21) der Kupplung (17) ausgebildeten Innengewindes (22) in den Spannabschnitt (21) einschraubbar ist, wobei bevorzugt die Endfixierstellung des Aufspreizelementes (59) durch ein bestimmtes Einschraubdrehmoment des Vortriebswerkzeugs vorgegeben ist.

22. Operationssystem nach Anspruch 20 oder 21,
**dadurch gekennzeichnet,**
**dass** das Auflageorgan (75) eine Auflagefläche (77) für die Unterseite (12) des Lagerkörpers (11) und einen durchgehenden Aufnahmekanal (79) für einen Spannabschnitt (21) der Kupplung (17) aufweist, über den zum einen der Spannabschnitt (21) für das Vortriebswerkzeug zugänglich ist und der zum anderen aufgrund seiner an den von einer Kreisform abweichenden Außenquerschnitt des Spannabschnitts (21) angepassten Innenquerschnittsform als Verdrehsicherung für die Kupplung (17) ausgebildet ist.

23. Operationssystem nach Anspruch 22,
**dadurch gekennzeichnet,**
**dass** die Auflagefläche (77) des Auflageorgans (75) gegenüber einer Längsachse (81) der Endfixiereinrichtung entsprechend der Neigung des Lagerkörpers (11) relativ zu einer Längsachse (33) der Kupplung (17) geneigt ist.

24. Operationssystem nach einem der Ansprüche 20 bis 23,
**dadurch gekennzeichnet,**
**dass** die Endfixiereinrichtung zwei durch mehrere Verbindungssäulen (83) in einem festen axialen Abstand gehaltene Grundplatten (85, 87) umfasst, wobei das Halteorgan (73) an der einen Grundplatte (85) und das Auflageorgan (75) an der anderen Grundplatte (87) abgestützt ist.

25. Operationssystem nach einem der vorhergehenden Ansprüche,
**dadurch gekennzeichnet,**
**dass** es ein Schlagwerkzeug (91) umfasst, mit dem auf den auf dem Kugellagerabschnitt (27) der Kupplung (17) angeordneten Lagerkörper (11) zum Einschlagen der Kupplung (17) in den Schaft (15) Schlagimpulse aufbringbar sind, wobei die Größe eines jeweils aufzubringenden Schlagimpulses am Schlagwerkzeug (91) voreinstellbar und insbesondere veränderbar ist.

## Claims

1. An operation system for a joint prosthesis, in particular a shoulder joint prosthesis, having two cooperating bearing bodies (11, 102), in particular a joint head (11) and a joint shell (102), a shaft (15) and a coupling (17) to connect the shaft (15) to one of the bearing bodies (11), **characterized by**
- a positioning device by means of which the shaft (15) can be positioned at a desired depth in the bone (19) without a coupling (17);
- a pre-fixing device by means of which the desired position of the bearing body (11) relative to the coupling (17) can be pre-fixed at the shaft (15) positioned at the desired depth; and
- a final fixing device by means of which, with the coupling (17) removed from the shaft (15), the pre-fixed desired position can be finally fixed.

2. An operation system in accordance with claim 1, **characterized in that** the coupling (17) for connection to the shaft (15) includes a clamping section (21) with which a firm clamped seating of the coupling (17) in the shaft (15) can be established by hammering into a coupling mount (23) of the shaft (15).

3. An operation system in accordance with claim 2, **characterized in that** the clamping section (21) tapers conically and can be hammered into a correspondingly shaped coupling mount (23) of the shaft (15), with the clamping section (21) preferably having an outer cross-section, in particular an elliptical outer cross-section, different from a circular shape.

4. An operation system in accordance with any one of the preceding claims, **characterized in that** the positioning device has a bearing section (31) fixable to the shaft and at least one positioning element (35), which is mounted pivotally and rotatably at the bearing section (31) and axially immovable in the direction of a longitudinal axis (33) relative to the bearing section (31), with a lower side (37) which faces the shaft (15), serves as a depth stop and can be brought into all orientations coming into question for the bearing body (11) relative to a prepared planar upper side (25) of the bone (19) by pivoting and/or rotating of the positioning element (35) relative to the bearing section (31).

5. An operation system in accordance with claim 4, **characterized in that** the bearing section (31) of the positioning device is made as a spherical bearing, with the center of the spherical bearing and the lower side (37) of the positioning element (35) being matched to one another with respect to their relative axial position such that, with the shaft (15) positioned at the desired depth and the bearing section (31) fixed to the shaft (15), the position of the center of the spherical bearing coincides with the desired position of the center of a spherical bearing section (27) of the coupling (17).

6. An operation system in accordance with claim 4 or claim 5, **characterized in that** the positioning element (35) corresponds at least approximately to the bearing body (11) with respect to the shape and to the size of its planar lower side (37) and to the position, in particular to the eccentric position, of a mount (39) opening at the lower side (37).

7. An operation system in accordance with any one of the claims 4 to 6, **characterized in that** the positioning element (35) is provided in the form of an annular disk.

8. An operation system in accordance with any one of the claims 4 to 7, **characterized in that** a plurality of positioning elements (35) are provided which differ from one another with respect to the shape and to the size of their planar lower sides (37) and to the position, in particular the eccentric position, of a mount (39) opening at the lower side (37).

9. An operation system in accordance with any one of the claims 4 to 8, **characterized in that** the positioning element (35) has at least one pressing screw which can be brought from a neutral position into a pressure position projecting from the lower side (37) of the positioning element (35) to press the positioning element (35) from the upper side (25) of the bone (19).

10. An operation system in accordance with any one of the claims 4 to 9, **characterized in that** the bearing section (31) of the positioning device has a coupling section (43) which is insertable into a coupling mount (23) of the shaft (15) and which has an outer shape matched to the inner cross-sectional shape of the coupling mount (23) differing from a circular shape to align the positioning device relative to the shaft (15).

11. An operation system in accordance with any one of the claims 4 to 10, **characterized in that** a transition is formed as a supporting and sealing area (45) between the bearing section (31) and the coupling section (43) of the positioning device, said supporting and sealing area contacting the shaft (15), when the bearing section (31) is fixed to the shaft (15), and sealing the interior of the shaft with respect to the environment.

12. An operation system in accordance with any one of the claims 4 to 11, **characterized in that** the bearing section (31) can be fixed to the shaft (15) by means of a clamping device (47) of the positioning device, with the clamping device (47) cooperating with an existing internal thread (29) formed in a coupling mount (23) of the shaft (15).

13. An operation system in accordance with any one of the claims 4 to 11, **characterized in that** the bearing section (31) of the positioning element is provided with a cylindrical surface region (49) to attach the positioning element (35).

14. An operation system in accordance with claim 13, **characterized in that** the center axis of the cylindrical surface region (49) has an inclination with respect to a longitudinal axis (33) of the bearing section (31) which is outside a zone of inclinations which the positioning element (35) can adopt during the shaft positioning.

15. An operation system in accordance with any one of the preceding claims, **characterized in that** the pre-fixing device includes a support member (51) which can be axially fixed in a coupling mount (23) of the shaft (15) and serves in the fixed state as an end stop for a clamping section (21) of the coupling (17) which can be secured in the coupling mount (23) by hammering in, with the support member (51) preferably being matched to the dimensions of the shaft (15) and of the clamping section (21) such that the support member (51) intercepts the clamping section (21) at a pre-fixing depth allowing a simple removal of the coupling (17) before reaching a desired depth required for the securing.

16. An operation system in accordance with claim 15, **characterized in that** the support member (51) can be axially fixed by screwing into the shaft (15) while utilizing an existing internal thread (29) formed in the coupling mount (23) of the shaft (15).

17. An operation system in accordance with claim 15 or claim 16, **characterized in that** the pre-fixing device includes a compensation member (53) which can be brought between the bone (19) and the bearing body (11) to compensate the difference between the desired depth and the pre-fixing depth and is made such that an alignment of the lower side (13) of the bearing body (11) parallel to the upper side (25) of the bone (19) is ensured.

18. An operation system in accordance with claim 17, **characterized in that** the compensation member (53) is provided in the form of a spring disk which is preferably made in one piece and/or preferably includes a plurality of spring tongues projecting from a base plate (55).

19. An operation system in accordance with any one of the preceding claims, **characterized in that** the pre-fixing device includes a spreading element (59), in particular a conical spreading element, which can be introduced into a spreadable spherical bearing section (27) of the coupling (17) and can be driven, for the pre-fixing spreading of the spherical bearing section (27), into said spherical bearing section (27) by hammering the coupling (17) into the shaft (15) - with the bearing body (11) arranged on its spherical bearing section (27) - by means of the support member (51) axially fixed in the shaft (15).

20. An operation system in accordance with any one of the preceding claims, **characterized in that** the final fixing unit includes a clamping apparatus (71) to clamp the bearing body (11) pre-fixed at the coupling (17) between a preferably axially adjustable holding member (73) and a preferably axially fixed supporting member (75) as well as a driving tool with which a spreading element (59) pre-fixing the bearing body (11) can be driven into a final fixing position to finally fix the bearing body (11) when the bearing body (11) is clamped in.

21. An operation system in accordance with claim 20, **characterized in that** the driving tool to drive in the spreading element (59) can be screwed into the clamping section (21) while using an existing internal thread (22) formed in a clamping section (21) of the coupling (17), with the final fixing position of the spreading element (59) preferably being pre-determined by a specific screw-in torque of the driving tool.

22. An operation system in accordance with claim 20 or claim 21, **characterized in that** the support member (75) has a supporting surface (77) for the lower side (12) of the bearing body (11) and a throughgoing receiving passage (79) for a clamping section (21) of the coupling (17) via which the clamping section (21) is accessible to the driving tool, on the one hand, and which is made as a rotational security for the coupling (17) due to its internal cross-section matched to the outer cross-section of the clamping section (21) differing from a circular shape, on the other hand.

23. An operation system in accordance with claim 22, **characterized in that** the support surface (77) of the support member (75) is inclined with respect to a longitudinal axis (81) of the final fixing device in accordance with the inclination of the bearing body (11) relative to a longitudinal axis (33) of the coupling (17).

24. An operation system in accordance with any one of the claims 20 to 23, **characterized in that** the final fixing device includes two base plates (85, 87) held at a fixed axial spacing by a plurality of connection columns (83), with the holding member (73) being supported at the one base plate (85) and the support member (75) being supported at the other base plate (87).

25. An operation system in accordance with any one of the preceding claims, **characterized in that** it includes a hammer tool (91) with which hammer impulses can be applied to the bearing body (11) arranged on the spherical bearing section (27) of the coupling (17) to hammer the coupling (17) into the shaft (15), with the magnitude of a hammer impulse to be applied in each case being pre-settable, and in particular changeable, at the hammer tool (91).

## Revendications

1. Système d'opération pour une prothèse d'articulation, en particulier une prothèse d'articulation d'épaule comportant deux corps de palier (11, 102), en particulier une tête d'articulation (11) et une coquille d'articulation (102), une tige (15) et un coupleur (17) pour relier la tige (15) à l'un des corps de palier (11), **caractérisé par**
- un dispositif de positionnement au moyen duquel la tige (15) peut être positionnée sans le coupleur (17) dans une profondeur de consigne dans l'os (19),
- un dispositif de fixation préalable au moyen duquel on peut fixer préalablement la position de consigne du corps de palier (11) par rapport au coupleur (17) sur la tige positionnée à la profondeur de consigne, et
- un dispositif de fixation définitive au moyen duquel on peut fixer définitivement la position de consigne préalablement fixée lorsque le coupleur (17) est enlevé de la tige (15).

2. Système d'opération selon la revendication 1,
**caractérisé en ce que**
pour être relié à la tige (15), le coupleur (17) comprend un tronçon de serrage (21) par lequel on peut réaliser un ajustement fermement serré du coupleur (17) dans la tige (15) en l'enfonçant dans un logement d'accouplement (23) de la tige (15).

3. Système d'opération selon la revendication 2,
**caractérisé en ce que**
le tronçon de serrage (21) se rétrécit en forme de cône et peut être enfoncé dans un logement d'accouplement (23) de la tige (15), formé de manière correspondante, le tronçon de serrage (21) présentant une section transversale extérieure différente d'une forme circulaire, en particulier elliptique.

4. Système d'opération selon l'une des revendications précédentes,
**caractérisé en ce que**
le dispositif de positionnement présente un tronçon de palier (31) qui peut être fixé sur la tige ainsi qu'au moins un élément de positionnement (35) monté en pivotement et en rotation sur le tronçon de palier (31), immobile axialement en direction d'un l'axe longitudinal (33) par rapport au tronçon de palier (31), avec une face inférieure (37) tournée vers la tige (15) et servant de butée de profondeur, face inférieure qui peut être amenée par pivotement et/ou rotation de l'élément de positionnement (35) par rapport au tronçon de palier (31) dans toutes les orientations entrant en ligne de compte pour le corps de palier (11) par rapport à une face supérieure (25) plane préparée de l'os (19).

5. Système d'opération selon la revendication 4,
**caractérisé en ce que**
le tronçon de palier (31) du dispositif de positionnement est réalisé sous forme de roulement à billes, le centre du roulement à billes et la face inférieure (37) de l'élément de positionnement (35) sont adaptés l'un à l'autre du point de vue de leur position relative axiale lorsque la tige (25) est positionnée à la profondeur de consigne et lorsque le tronçon de palier (31) est fixé sur la tige (15), la position du centre du roulement à billes coïncide avec la position de consigne du centre d'un tronçon de roulement à billes (27) du coupleur (17).

6. Système d'opération selon la revendication 4 ou 5,
**caractérisé en ce que**
l'élément de positionnement (35) correspond au moins approximativement au corps de palier (11), du point de vue de la forme et de la taille de sa face inférieure (37) plane ainsi que du point de vue de la position en particulier excentrique d'un logement (39) débouchant sur la face inférieure (37).

7. Système d'opération selon l'une des revendications 4 à 6,
**caractérisé en ce que**
l'élément de positionnement (35) est prévu sous forme d'un disque annulaire.

8. Système d'opération selon l'une des revendications 4 à 7,
**caractérisé en ce que**
il est prévu une pluralité d'éléments de positionnement (35) qui se distinguent les uns des autres du point de vue de la forme et de la taille de leurs faces inférieures (37) planes, ainsi que du point de vue de la position en particulier excentrique d'un logement (39) débouchant sur la face inférieure (37).

9. Système d'opération selon l'une des revendications 4 à 8,
**caractérisé en ce que**
l'élément de positionnement (35) présente au moins une vis d'éjection qui peut être amenée depuis une position neutre jusque dans une position d'éjection faisant saillie depuis la face inférieure (37) de l'élément de positionnement (35), pour éjecter l'élément de positionnement (35) hors de la face supérieure (25) de l'os (19).

10. Système d'opération selon l'une des revendications 4 à 9,
**caractérisé en ce que**
le tronçon de palier (31) du dispositif de positionnement présente un tronçon d'accouplement (43) qui peut être introduit dans un logement d'accouplement (23) de la tige (15), lequel, pour aligner le dispositif de positionnement par rapport à la tige (15), présente une forme extérieure adaptée à la forme de section transversale intérieure du logement d'accouplement (23), laquelle diffère d'une forme circulaire.

11. Système d'opération selon l'une des revendications 4 à 10,
**caractérisé en ce que**
une transition est réalisée entre le tronçon de palier (31) et le tronçon d'accouplement (43) du dispositif de positionnement, laquelle sert de surface d'appui et d'étanchéité (45) qui est en appui sur la tige (15) lorsque le tronçon de palier (31) est fixé sur la tige (15) et qui rend étanche l'intérieur de la tige vis-à-vis de l'environnement.

12. Système d'opération selon l'une des revendications 4 à 11,
**caractérisé en ce que**
le tronçon de palier (31) peut être fixé sur la tige au moyen d'un dispositif de serrage (47) du dispositif de positionnement, le dispositif de serrage (47) coopérant avec un taraudage (29) réalisé dans un logement d'accouplement (23) de la tige (15).

13. Système d'opération selon l'une des revendications 4 à 11,
**caractérisé en ce que**
le tronçon de palier (31) du dispositif de positionnement est pourvu d'une région de surface (49) cylindrique pour monter l'élément de positionnement (35).

14. Système d'opération selon la revendication 13,
**caractérisé en ce que**
l'axe médian de la région de surface (49) cylindrique présente par rapport à un axe longitudinal (33) du tronçon de palier (31) une inclinaison qui est située en dehors d'une plage d'inclinaisons que peut occuper l'élément de positionnement (35) pendant le positionnement de la tige.

15. Système d'opération selon l'une des revendications précédentes,
**caractérisé en ce que**
le dispositif de fixation préalable comprend un organe de soutien (51) qui peut être fixé axialement dans un logement d'accouplement (23) de la tige (15) et qui, à l'état fixé, sert de butée d'extrémité pour un tronçon de serrage (21) du coupleur (17), lequel peut être fixé par enfoncement dans le logement d'accouplement (23), l'organe de soutien (51) étant de préférence adapté aux dimensions de la tige (15) et du tronçon de serrage (21) de telle sorte que l'organe de soutien (51) intercepte le tronçon de serrage (21) avant d'avoir atteint une profondeur de consigne nécessaire à la fixation à une profondeur de fixation préalable permettant d'enlever aisément le coupleur (17).

16. Système d'opération selon la revendication 15,
**caractérisé en ce que**
l'organe de soutien (51) peut être fixé axialement par vissage dans la tige (15) en utilisant un taraudage (29) existant réalisé dans le logement d'accouplement (23) de la tige (15).

17. Système d'opération selon la revendication 15 ou 16,
**caractérisé en ce que**
le dispositif de fixation préalable comprend un organe de compensation (53) qui peut être amené entre l'os (19) et le corps de palier (11) pour compenser la différence entre la profondeur de consigne et la profondeur de fixation préalable et qui est réalisé de manière à assurer un alignement de la face inférieure (13) du corps de palier (11) parallèlement à la face supérieure (25) de l'os (19).

18. Système d'opération selon la revendication 17,
**caractérisé en ce que**
l'organe de compensation (53) est prévu sous forme d'une rondelle élastique qui est réalisée de préférence d'un seul tenant et/ou qui comprend de préférence plusieurs languettes élastiques (57) faisant saillie depuis une plaque de base (55).

19. Système d'opération selon l'une des revendications précédentes,
**caractérisé en ce que**
le dispositif de fixation préalable comprend un élément d'expansion (59) en particulier conique qui peut être introduit dans un tronçon de roulement à billes (27) expansible du coupleur (17) et qui peut être chassé dans le tronçon de roulement à billes (27) par enfoncement du coupleur (17) dans la tige (15), lorsque le corps de palier (11) est agencé sur son tronçon de roulement à billes (27), au moyen de l'organe de soutien (51) fixé axialement dans la tige (15), pour soumettre le tronçon de roulement à billes (27) à une expansion de fixation préalable.

20. Système d'opération selon l'une des revendications précédentes,
**caractérisé en ce que**
le dispositif de fixation définitive comprend un dispositif de serrage (71) pour serrer le corps de palier (11) fixé préalablement sur le coupleur (17) entre un organe de retenue (73) de préférence axialement réglable et un organe d'appui (11) de préférence axialement fixe, ainsi qu'un outil d'avancement avec lequel, lorsque le corps de palier (11) est serré, on peut enfoncer jusque dans une position de fixation définitive un élément d'expansion (59) fixé préalablement dans le corps de palier (11) pour fixer définitivement le corps de palier (11).

21. Système d'opération selon la revendication 20,
**caractérisé en ce que**
l'outil d'avancement peut être vissé dans le tronçon de serrage (21) pour enfoncer l'élément d'expansion (59) en utilisant un taraudage (22) réalisé dans un tronçon de serrage (21) du coupleur (17), la position de fixation définitive de l'élément d'expansion (59) étant de préférence prédéterminée par un couple de rotation de vissage de l'outil d'avancement.

22. Système d'opération selon la revendication 20 ou 21,
**caractérisé en ce que**
l'organe d'appui (75) présente une surface d'appui (77) pour la face inférieure (12) du corps de palier (11) et un canal de réception (79) continu pour un tronçon de serrage (21) du coupleur (17), via lequel d'une part le tronçon de serrage (21) est accessible pour l'outil d'avancement et qui, d'autre part, est réalisé en tant que sécurité antirotation pour le coupleur (17) en raison de sa forme de section transversale intérieure adaptée à la section transversale extérieure du tronçon de serrage (21), laquelle diffère d'une forme circulaire.

23. Système d'opération selon la revendication 22,
**caractérisé en ce que**
la surface d'appui (77) de l'organe d'appui (75) est inclinée par rapport à un axe longitudinal (33) du coupleur (17) vis-à-vis d'un axe longitudinal (81) du dispositif de fixation définitive selon l'inclinaison du corps de palier (11).

24. Système d'opération selon l'une des revendications 20 à 23,
**caractérisé en ce que**
le dispositif de fixation définitive comprend deux plaques de base (85, 87) retenues par plusieurs colonnes de liaison (83) à une distance axiale fixe, l'organe de retenue (73) étant soutenu sur une des plaques de base (85) et l'organe d'appui (75) étant soutenu sur l'autre plaque de base (87).

25. Système d'opération selon l'une des revendications précédentes,
**caractérisé en ce que**
il comprend un outil de percussion (91) avec lequel on peut appliquer des impulsions de percussion sur le corps de palier (11) agencé sur le tronçon de roulement à billes (27) du coupleur (17) pour enfoncer le coupleur (17) dans la tige (15), la grandeur d'une impulsion de percussion respective à appliquer sur l'outil de percussion (91) pouvant être réglée à l'avance et étant en particulier variable.
